(19) Europäisches Patentamt
European Patent Office
Office européen des brevets

(11) EP 4 130 087 A1

(12) **EUROPEAN PATENT APPLICATION**
published in accordance with Art. 153(4) EPC

(43) Date of publication:
**08.02.2023 Bulletin 2023/06**

(21) Application number: **21776252.5**

(22) Date of filing: **25.03.2021**

(51) International Patent Classification (IPC):
***C08G 59/02*** *(1974.07)* ***C08G 59/18*** *(1974.07)*
***C07D 493/04*** *(1974.07)*

(52) Cooperative Patent Classification (CPC):
**C07D 493/04; C08G 59/02; C08G 59/18**

(86) International application number:
**PCT/JP2021/012523**

(87) International publication number:
**WO 2021/193819 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.03.2020 JP 2020058925**

(71) Applicant: **ENEOS Corporation**
**Chiyoda-ku**
**Tokyo 100-8162 (JP)**

(72) Inventors:
- **TAKATA, Shohei**
**Tokyo 100-8162 (JP)**
- **KOIKE, Takeshi**
**Tokyo 100-8162 (JP)**
- **UE, Kenta**
**Tokyo 100-8162 (JP)**

(74) Representative: **Müller-Boré & Partner Patentanwälte PartG mbB**
**Friedenheimer Brücke 21**
**80639 München (DE)**

(54) **STEREOISOMER OF EPOXY COMPOUND, CURABLE COMPOSITION CONTAINING SAME, AND CURED OBJECT OBTAINED BY CURING CURABLE COMPOSITION**

(57) Provided are a stereoisomer of an epoxy compound that when contained in a curable composition, can improve the heat resistance and dielectric properties of the cured product of the curable composition, a curable composition containing the stereoisomer, and a cured product of the curable composition. A stereoisomer of an epoxy compound represented by Formula (1) below, the stereoisomer being represented by Formula (2), and a curable composition containing the stereoisomer are used:

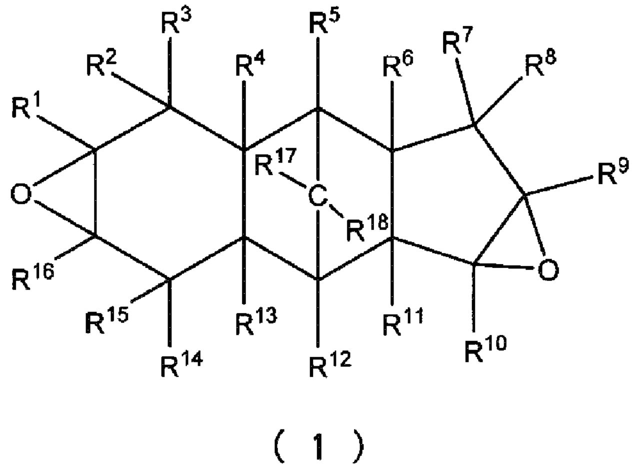

( 1 )

in which in Formula (1), $R^1$ to $R^{18}$ are each independently selected from the group consisting of hydrogen, an alkyl

EP 4 130 087 A1

group, and an alkoxy group; and

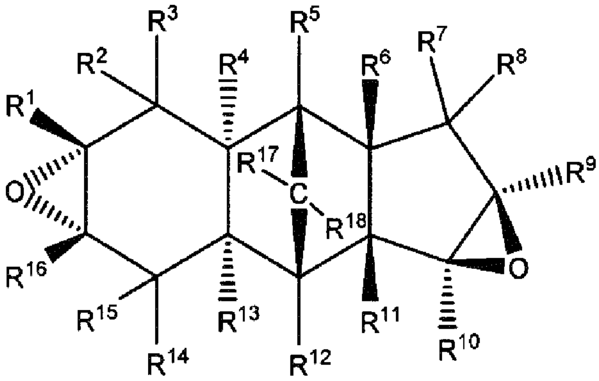

( 2 )

in which in Formula (2), $R^1$ to $R^{18}$ are each the same as in Formula (1).

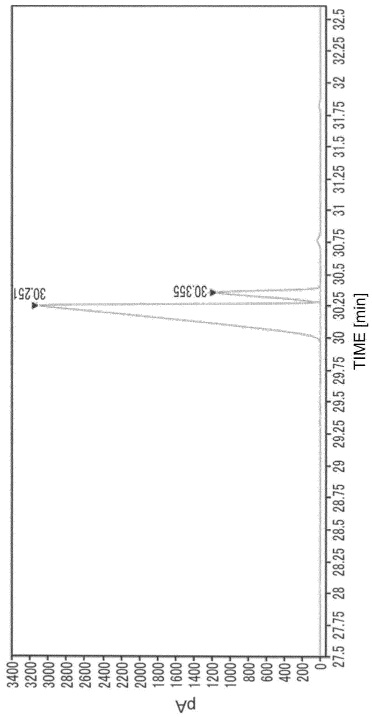


FIG. 1

## Description

### Technical Field

The present invention relates to stereoisomers of an epoxy compound, a curable composition containing the same, and a cured product obtained by a curing curable composition.

### Background Art

Curable compositions containing epoxy compounds are used as materials for surface protective films, interlayer insulators, protective insulating films for print-oriented substrates, and fiber-reinforced composite materials of semiconductor devices and organic thin film devices (for example, organic electroluminescent devices and organic thin-film solar cells). Among these epoxy compounds, epoxy compounds having an aromatic ring have been used for obtaining cured products excellent in heat resistance or the like.

However, compounds having an aromatic ring have caused problems in applications as described above in the electronic material field due to their commonly high electron density and high dielectric constant. Furthermore, recent advances in the miniaturization and higher performance of electronic devices have been accompanied by improved performance requirements for the various materials used. For example, as signals become faster and have higher frequency, materials with low electrical energy loss, i.e., materials with excellent dielectric characteristics, are required. Thus, in recent years, alicyclic diamine compounds having no aromatic ring have been drawing attention. In addition, curable compositions used in the applications as described above are required to give cured products having high heat resistance.

Among these epoxy compounds, epoxy compounds having an alicyclic skeleton have been known as those that give cured products excellent in heat resistance or the like. For example, Patent Document 1 discloses an epoxy compound having an alicyclic skeleton having a specific structure, which enables obtaining resins having excellent heat resistance or the like.

Among these epoxy compounds, epoxy compounds having two or more alicyclic skeletons in their molecules have been known as those that give cured products excellent in heat resistance, transparency, or the like. For example, Patent Document 2 discloses a curable composition containing a diepoxybicyclohexyl compound.

Meanwhile, in recent years, more highly heat-resistant members have been demanded in fields such as surface protective films, interlayer insulators, and protective insulating films for print-oriented substrates of semiconductor devices and organic thin film devices as described above. In addition, as described above, materials having excellent dielectric characteristics have also been demanded in the electronic material field. Therefore, conventional epoxy compounds as proposed in Patent Document 2 have had a room for further improvement from the viewpoint of heat resistance, dielectric properties, and the like of cured products.

### Prior Art References

#### Patent Documents

Patent Document 1: JP-A No. S49-126658
Patent Document 2: JP-A No. 2008-31424

## SUMMARY OF THE INVENTION

### Problems to be Solved by the Invention

The present inventors have now found that a mixture of stereoisomers of an epoxy compound, comprising a stereoisomer having a specific structure in a certain proportion or more can dramatically improve the heat resistance and dielectric properties of the cured product obtained by curing a curable composition comprising the mixture.

Therefore, an object of the present invention is to provide a stereoisomer of an epoxy compound that when contained in a curable composition can improve the heat resistance and dielectric properties of the cured product. In addition, another object of the present invention is to provide a curable composition for obtaining a cured product having excellent heat resistance and dielectric properties.

Means for Solving the Problems

Accordingly, the present invention includes the following inventions:

[1] A stereoisomer of an epoxy compound represented by Formula (1) below:

the stereoisomer being represented by Formula (2):

[Chem 1]

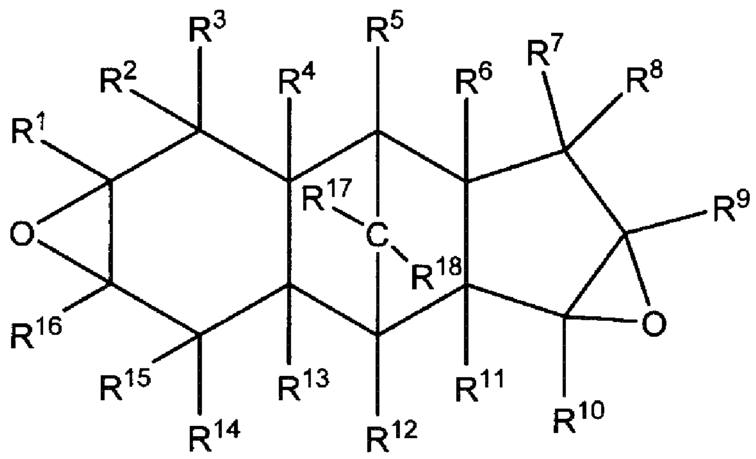

(1)

wherein, in Formula (1),
$R^1$ to $R^{18}$ are each independently selected from the group consisting of hydrogen, an alkyl group, and an alkoxy group; and

[Chem 2]

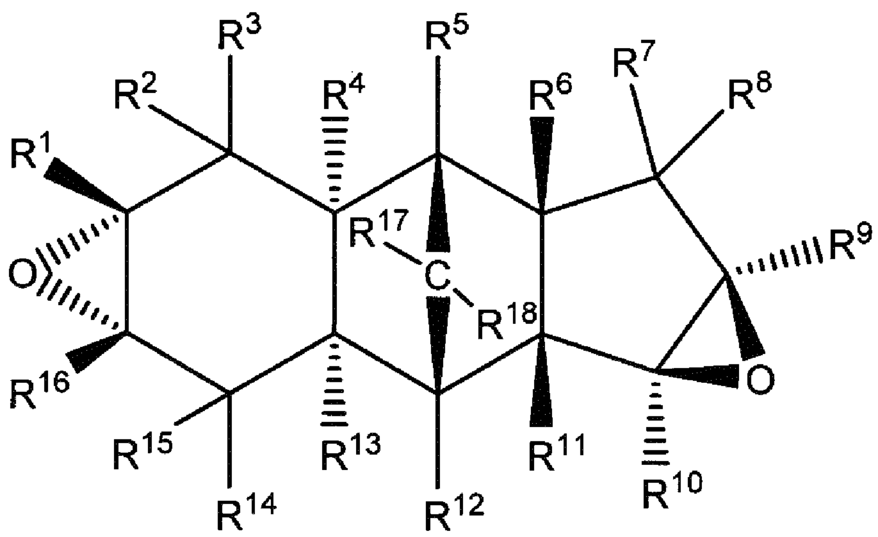

(2)

wherein, in Formula (2),
$R^1$ to $R^{18}$ are each the same as in Formula (1).

[2] The stereoisomer according to [1], wherein $R^1$ to $R^{18}$ are all hydrogen.
[3] A mixture of stereoisomers of a compound represented by Formula (1) above,
wherein the amount of the stereoisomer according to [1] or [2] relative to the total amount of the stereoisomer mixture is 60 mol% or more.
[4] A curable composition comprising the stereoisomer according to [1] or [2] or the mixture according to [3].
[5] The curable composition according to [4], further comprising at least one selected from the group consisting of a curing agent, a thermal cationic polymerization initiator, and a photo-cationic polymerization initiator.
[6] The curable composition according to [5], wherein the curing agent is at least one selected from an acid anhydride-based compound and an anion-based compound.
[7] The curable composition according to [6], wherein the anion-based compound is at least one selected from the group consisting of an amine-based compound, a phenol-based compound, a thiol-based compound, and a latent curing agent.
[8] The curable composition according to any one of [4] to [7], further comprising a curing accelerator.
[9] The curable composition according to [8], wherein the curing accelerator is an imidazole-based curing accelerator.
[10] The curable composition according to any one of [4] to [9], further comprising at least one selected from the group consisting of an epoxy compound other than the compound represented by Formula (1), an oxetane compound, and vinyl ether.
[11] A cured product obtained by curing the curable composition according to any one of [4] to [10].
[12] A method of producing a cured product, comprising a step of curing the curable composition according to any one of [4] to [10].

Effects of the Invention

According to the present invention, a stereoisomer or a mixture of stereoisomers that can be used to produce cured products having excellent heat resistance and dielectric properties can be provided. In other words, according to the present invention, a stereoisomer represented by Formula (2) above, or a mixture of stereoisomers of a compound represented by Formula (1), comprising the stereoisomer represented by Formula (2) in a specific ratio or more can be used as an agent for improving the heat resistance and dielectric properties. In addition, according to the present invention, a curable composition for obtaining a cured product that is excellent in heat resistance and dielectric properties can be provided.

BRIEF DESCRIPTION OF THE DRAWINGS

FIG. 1 represents gas chromatograph of an epoxy compound (A-1) synthesized in Preparation Example 1.
FIG. 2 represents a $^1$H NMR peak chart of a sample obtained in Preparation Example 2.
FIG. 3 represents a $^{13}$C NMR peak chart of a sample obtained in Preparation Example 2.
FIG. 4 represents a NOESY spectrum graph of a sample obtained in Preparation Example 2.
FIG. 5 represents a graph enlarging a part of the NOESY spectrum graph of FIG. 4.

DETAILED DESCRIPTION OF THE INVENTION

Definition

Unless otherwise specified herein, the terms "part," "%," and the like are based on mass. As used herein, the term "epoxy equivalent" is defined as the mass of an epoxy compound having 1 equivalent of an epoxy group. Here, in the case of a mixture composed of m types (m is an integer of 2 or more) of epoxy compounds, the epoxy equivalent of the mixture is represented as:

[math 1]

$$\text{EPOXY EQUIVALENT OF MIXTURE OF EPOXY COMPOUNDS} = \frac{\sum_{n=1}^{m} \text{MASS OF EPOXY COMPOUND N}}{\sum_{n=1}^{m} \frac{\text{MASS OF EPOXY COMPOUND N}}{\text{EPOXY EQUIVALENT OF EPOXY COMPOUND N}}}$$

The epoxy equivalent of an epoxy compound can be measured according to JIS K7236.

Stereoisomer of Epoxy Compound

In one embodiment of the present invention, the stereoisomer of an epoxy compound is a stereoisomer of an epoxy compound represented by Formula (1) below, the stereoisomer being represented by the following Formula (2). The stereoisomer represented by the following Formula (2) can be contained in a curable composition.

[Chem 3]

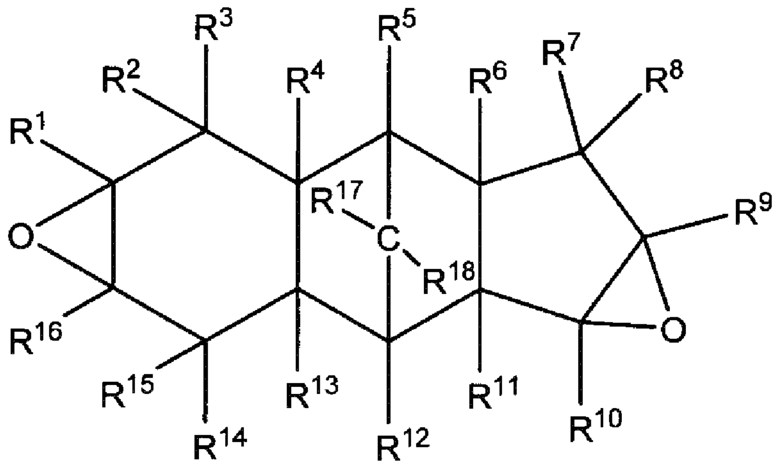

( 1 )

In Formula (1), $R^1$ to $R^{18}$ are each independently selected from the group consisting of hydrogen, an alkyl group, and an alkoxy group.

[Chem 4]

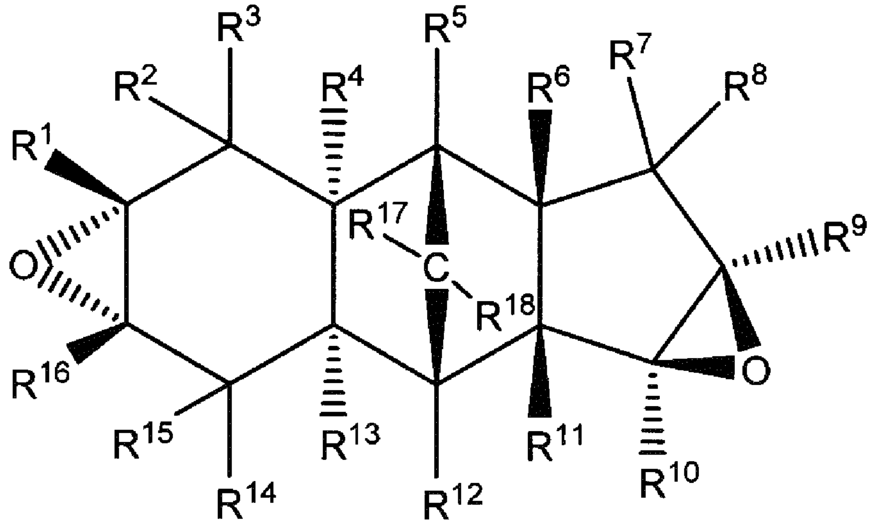

( 2 )

In Formula (2), $R^1$ to $R^{18}$ are each independently selected from the group consisting of hydrogen, an alkyl group, and an alkoxy group.

In one embodiment of the present invention, in the epoxy compound of the present invention or the stereoisomer of the present invention, $R^1$ to $R^{18}$, in Formula (1) or (2) above, are each independently selected from the group consisting of hydrogen, an alkyl group, and an alkoxy group. Among them, hydrogen is particularly preferable.

The carbon number of the alkyl group is preferably from 1 to 10, and more preferably from 1 to 4. The alkyl group may be a linear or branched-chain alkyl group.

Examples of the linear alkyl group include a methyl group, an ethyl group, a n-propyl group, and a n-butyl group.

Examples of the branched-chain alkyl group include an i-propyl group, an i-butyl group, a s-butyl group, and a t-butyl group.

The carbon number of the alkoxy group is preferably from 1 to 10, and more preferably from 1 to 4.

The alkyl group that is a part of the alkoxy group may be a linear or branched-chain alkyl group.

Examples of the alkoxy group include a methoxy group, an ethoxy group, a propoxy group, an i-propoxy group, a n-butoxy group, an i-butoxy group, a s-butoxy group, and a t-butoxy group.

In preferred embodiments of the present invention, $R^1$ to $R^{18}$, in Formula (1) and/or (2) above, are all hydrogen.

In preferred embodiments of the present invention, $R^1$ to $R^{18}$ in Formula (2) above are the same as $R^1$ to $R^{18}$ in Formula (1) above, respectively.

In one embodiment of the present invention, the mixture of stereoisomers of a compound represented by Formula (1) above is a mixture in which the amount of the stereoisomer represented by Formula (2) above is 60 mol% or more relative to the total amount of the stereoisomer mixture. The amount is preferably 70 mol% or more, and more preferably 75 mol% or more.

In other embodiments of the present invention, the mixture of stereoisomers of a compound represented by Formula (1) above is a mixture comprising at least a stereoisomer represented by Formula (2) above of stereoisomers of a compound represented by Formula (1) above, wherein, in the gas chromatogram obtained by gas chromatography analysis of the mixture under the following analysis conditions, the proportion of the peak area of the maximum peak is 60% or more relative to the sum of the peak areas derived from the stereoisomers of a compound represented by Formula (1), and the peak top of the maximum peak exists in the retention time range of 29.80 to 30.30 minutes. The mixture of the present invention is a mixture comprising at least a stereoisomer represented by Formula (2) above of stereoisomers of a compound represented by Formula (1) above, wherein in the gas chromatogram obtained by gas chromatography analysis of the mixture under the following analysis conditions, the proportion of the peak area of the maximum peak having the peak top in the retention time range of 29.90 to 30.30 minutes relative to the sum of the peak areas of the peaks in the retention time range of 29.80 to 30.50 minutes is 60% or more.

(Analysis Conditions)

Column: HP-5MS (Agilent Technologies, Inc.), length: 30.0 m, inner diameter: 320 μm, film thickness: 250 μm
Liquid phase: (5%-phenyl)-methylpolysiloxane
Carrier gas: $N_2$
Flow rate: 5.2 mL/min
Split ratio: 60:1
Temperature of sample inlet: 200°C
Temperature of detector: 250°C
Column temperature rising condition: 50°C (5 minutes), 50 to 150°C (5°C/min), 150 to 250°C (10°C/min), 250°C (10 minutes)

In other embodiments of the present invention, the mixture of stereoisomers of a compound represented by Formula (1) above is a mixture comprising at least a stereoisomer represented by Formula (2) above of stereoisomers of a compound represented by Formula (1) above, which is, in the gas chromatogram obtained by gas chromatography analysis of the mixture under the analysis conditions described above, derived from stereoisomers of a compound represented by Formula (1).

In other embodiments of the present invention, the mixture of stereoisomers of a compound represented by Formula (1) above shows a proportion of the peak area of the maximum peak of 60% or more relative to the sum of the peak areas derived from the stereoisomers of a compound represented by Formula (1) contained in the mixture in the gas chromatogram obtained by gas chromatography analysis under the analysis conditions described above. The proportion of the peak area of the maximum peak is preferably 62% or more, more preferably 65% or more, still more preferably 70% or more, and even still more preferably 75% or more. The peak top of the maximum peak is present in the retention time range of 29.80 to 30.30.

In another embodiment of the present invention, the mixture is a mixture comprising at least a stereoisomer represented by Formula (2) above of stereoisomers of a compound represented by Formula (1) above, wherein in the gas chromatogram obtained by gas chromatography analysis of the mixture under the analysis conditions described above, the proportion of the peak area of the maximum peak having the peak top in the retention time range of 29.90 to 30.30 minutes relative to the sum of the peak areas of the peaks in the retention time range of 29.80 to 30.50 minutes is 60% or more. The proportion of the peak area of the maximum peak is preferably 65% or more, more preferably 70% or more, and still more preferably 75% or more.

In one embodiment of the present invention, the stereoisomer corresponding to the maximum peak described above in the mixture of stereoisomers of a compound represented by Formula (1) above is preferably represented by Formula (2) above.

Method of Producing Stereoisomer of Epoxy Compound

The method of producing stereoisomers represented by Formula (2), which are stereoisomers of an epoxy compound represented by Formula (1) will be described in detail.

Stereoisomers represented by Formula (2) can be obtained by a method comprising a step of reacting a compound represented by the following Formula (3) and a peroxy acid:

[Chem 5]

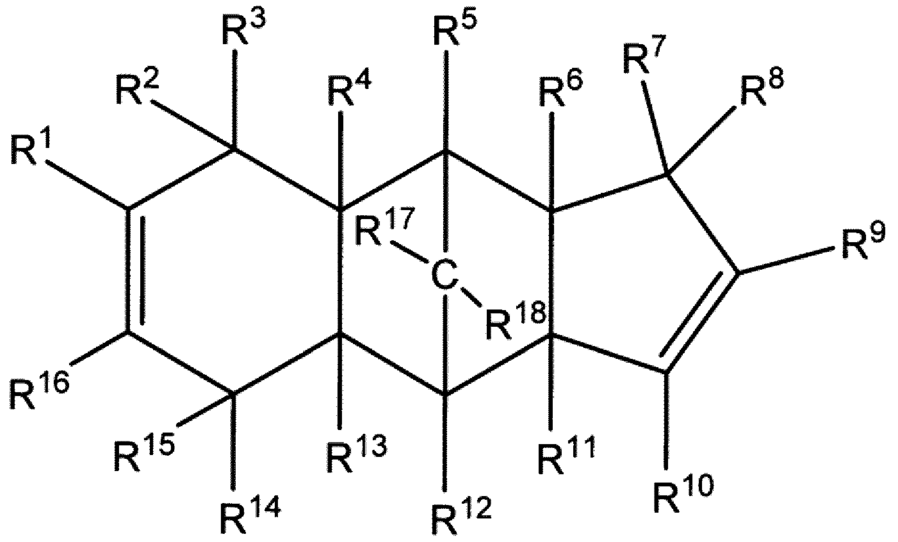

( 3 )

wherein $R^1$ to $R^{18}$ are each independently selected from the group consisting of hydrogen, an alkyl group, and an alkoxy group.

$R^1$ to $R^{18}$ in Formula (3) above are as described for Formula (1).

In one embodiment of the present invention, the stereoisomers represented by Formula (2) above can be synthesized by reacting a compound represented by Formula (3) above and a peroxy acid such as hydrogen peroxide, performic acid, peracetic acid, or perbenzoic acid.

In one embodiment of the present invention, the compound represented by Formula (3) above can be synthesized by the Diels-Alder reaction between butadiene and dicyclopentadiene.

In one embodiment of the present invention, production of stereoisomers represented by Formula (2) above when hydrogen peroxide is used as the peroxy acid is performed preferably in the presence of a catalyst and/or a surfactant, and more preferably in the presence of a catalyst and a surfactant.

Examples of the catalyst include tungsten compounds, phosphates, phosphonates, and salts thereof.

The tungsten compounds are not particularly limited as long as they can produce a tungstate anion in water and catalyze the epoxidation reaction of carbon-carbon double bonds by hydrogen peroxide. Examples include tungstic acid, tungstic trioxide, tungsten trisulfide, tungsten hexachloride, phosphotungstic acid, silicotungstic acid, and the like; and tungstates such as ammonium tungstate, potassium tungstate, sodium tungstate, and calcium tungstate. Among them, tungstic acid, tungstic trioxide, phosphotungstic acid, and sodium tungstate are preferable, and sodium tungstate dihydrate is more preferable. These tungsten compounds may be used alone or in combination of two or more thereof.

The phosphates, phosphonates, and salts thereof are not particularly limited as long as they can catalyst the epoxidation reaction of carbon-carbon double bonds by hydrogen peroxide. Examples of phosphoric acid include phosphoric acid, polyphosphoric acid, pyrophosphate, hexametaphosphic acid, hypophosphite, phosphorous acid, dodecylphosphoric acid, 2-ethylhexylphosphoric acid, and the like. Examples of the salt of the phosphoric acid include sodium phosphate, potassium phosphate, ammonium phosphate, sodium dihydrogen phosphate, disodium hydrogen phosphate, potassium hydrogen phosphate, ammonium hydrogen phosphate, sodium polyphosphate, sodium hexametaphosphate, acidic sodium hexametaphosphate, sodium polyphosphate, sodium pyrophosphate, disodium dihydrogen pyrophosphate, sodium hypophosphite, and sodium phosphite. Examples of the phosphonic acid include methylphosphonic acid, ethylphosphonic acid, n-propylphosphonic acid, isopropylphosphonic acid, n-butylphosphonic acid, t-butylphosphonic acid, phenylphosphonic acid, 4-methoxyphenylphosphonic acid, 4-aminophenylphosphonic acid, 1-hydroxyethane-1,1-bis(phosphonic acid), nitrilotris (methylenephosphonic acid), and the like. Examples of the salt of the phosphonic acid include sodium phenylphosphonate and the like. Among these, from the viewpoint of availability and reaction activity, phosphoric acid, phenylphosphonic acid, phosphorous acid, hypophosphorous acid, 2-ethylhexylphosphoric acid, lauryl phosphoric acid, sodium dihydrogen phosphate, and the like are preferred, and among them, phenylphosphonic acid is

more preferred. In the present invention, a single one or a combination of two or more selected from the group consisting of the phosphates, phosphonates, and salts thereof described above can be used.

The amount of the catalyst used is usually from 0.0001 to 20 mol%, and preferably from 0.01 to 10 mol%, relative to 1 mol of the compound of Formula (3). When the catalyst is a combination of a plurality of catalysts, the amount of the catalysts used means the total amount.

Examples of the surfactant include ionic surfactants. Examples of the ionic surfactants include cationic surfactants and anionic surfactants. Examples of the cationic surfactants include quaternary ammonium salts, nitrogen ring-containing quaternary ammonium salts, quaternary phosphonium salts, and quaternary sulfonium salts, and quaternary ammonium salts are especially preferable. Examples of the quaternary ammonium cation includeBenzyltrimethylammonium, benzyltributylammonium, benzyltrimethylammonium, lauryldimethylbenzylammonium, tetramethylammonium, tetra propylammonium, tetrabutylammonium, tetrahexylammonium, tetraoctylammonium, trioctylethylammonium, dilauryldimethylammonium, didecyldiethylammonium, didecyldipropylammonium, dioleyldimethylammonium, lauryltrimethylammonium, distearyldimethylammonium,stearyltrimethylammonium, dioctadecyldimethylammonium, dicetyldimethylammonium, cetyltrimethylammonium, tricaprylmethylammonium, palmityldimethylethylammonium, hexadecyltrimethylammonium, and lauryldimethylethylammonium. Preferred quaternary ammonium salts are hexadecyltrimethylammonium methyl sulfate, trioctylmethyl methyl sulfate, and didecyldimethyl methyl sulfate. The amount of the surfactant used is usually from 0.0001 to 10 mol%, and preferably from 0.01 to 10 mol%, relative to 1 mol of the compound of Formula (3).

In one embodiment of the present invention, crystallization purification can further increase the proportion of stereoisomers represented by Formula (2) above in the mixture. More specifically, the proportion of stereoisomers represented by Formula (2) above can be increased by dissolution of the mixture in isopropyl alcohol, cooling to -10°C, crystallization, and then filtration, followed by drying of the obtained residue.

In one embodiment of the present invention, crystallization purification can further increase the proportion of the proportion of the peak area of the maximum peak having the peak top in the retention time range of 29.90 to 30.30 in the gas chromatogram obtained by gas chromatography analysis under the analysis conditions described above. More specifically, the proportion of the peak area of the maximum peak can be increased by dissolution of the mixture in isopropyl alcohol, cooling to -10°C, crystallization, and then filtration, followed by drying of the obtained residue.

Curable Composition

In one embodiment of the present invention, a curable composition comprises stereoisomers represented by Formula (2) above or a mixture of stereoisomers of a compound represented by Formula (1) above.

The curable composition can contain stereoisomers represented by Formula (2) above or a mixture of stereoisomers of a compound represented by Formula (1) above, and optionally, from the viewpoint of more effectively imparting heat resistance and/or dielectric properties to the cured product, other components (for example, curing agents, thermal cationic polymerization initiators, photo-cationic polymerization initiators, curing accelerators, epoxy compounds other than the compound represented by Formula (1) above, oxetane compounds, and vinyl ether) in combination.

Components that can be contained in the curable composition will be described in detail below.

Stereoisomers Represented by Formula (2) or Mixture of Stereoisomers of Compound Represented by Formula (1)

Stereoisomers represented by Formula (2) or a mixture of stereoisomers of a compound represented by Formula (1) is/are as described above.

In one embodiment of the present invention, the mixing ratio of the mixture of stereoisomers of a compound represented by Formula (1) in the curable composition, relative to 100 parts by mass of the curable composition in total, is preferably in the range of 5 to 99 parts by mass, more preferably in the range of 10 to 80 parts by mass, and still more preferably in the range of 15 to 40 parts by mass. In other embodiments of the present invention, the mixing ratio of stereoisomers represented by Formula (2) in the curable composition, relative to 100 parts by mass of the curable composition in total, is preferably in the range of 5 to 80 parts by mass, more preferably in the range of 10 to 50 parts by mass, and still more preferably in the range of 12 to 40 parts by mass. Inclusion of stereoisomers represented by Formula (2) or a mixture of stereoisomers of a compound represented by Formula (1) within the range enables obtaining a cured product having more excellent heat resistance and dielectric properties.

In one embodiment of the present invention, when a curable composition comprising stereoisomers represented by Formula (2) of the present invention or a mixture of stereoisomers of a compound represented by Formula (1), and an acid anhydride-based compound, or a curable composition comprising stereoisomers represented by Formula (2) or a mixture of stereoisomers of a compound represented by Formula (1) of the present invention, an acid anhydride-based compound, and a curing accelerator, the stereoisomers of Formula (2) or the mixture of stereoisomers of a compound of Formula (1) contained in the curable composition have/has an epoxy equivalent of preferably from 70 to 600 g/eq, more preferably from 70 to 400 g/eq, still more preferably from 85 to 300 g/eq, still more preferably from 90 to 300 g/eq,

and still more preferably from 90 to 200 g/eq.

Curing Agent

In one embodiment of the present invention, the curing agent that can be contained in the curable composition may be, for example, an acid anhydride-based compound or an anion-based compound (for example, an amine-based compound, a phenol-based compound, or a latent curing agent), and preferably is an acid anhydride-based compound or a phenol-based compound.

Acid Anhydride-based Compound

As the acid anhydride type compound contained in the curable composition of the present invention, hexahydrophthalic anhydride, methyl hexahydrophthalic anhydride, tetrahydrophthalic anhydride, methyl tetrahydrophthalic anhydride, endomethylene tetrahydrophthalic anhydride, methyl butenyl tetrahydrophthalic anhydride, hydrogenated methyl nadic anhydride, cyclohexane dicarboxylic anhydride, methyl cyclohexane tetracarboxylic anhydride, maleic anhydride, phthalic anhydride, dodecenyl succinic anhydride, octenyl succinic anhydride, pyromellitic acid,trimellitic anhydride, alkyl styrenemaleic anhydride copolymer, chlorendoic anhydride, polyazelaic anhydride, benzophenone tetracarboxylic anhydride, ethylene glycol bisanhydrotrimellitate, Glycerol tritrimellitate, glycerin bis (anhydrotrimellitate) monoacetate, benzophenone tetracarboxylic acid, polyadipic acid anhydride, polysebacic acid anhydride, poly (ethyloctadecanedioic acid) anhydride, poly (phenylhexadecanedio ic acid) anhydride, Hett acid anhydride, norbornane-2,3-dicar boxylic acid anhydride, and the like can be mentioned.

Among them, hexahydrophthalic anhydride, methylhexahydrophthalic anhydride, or a combination thereof is preferable because of it can further improve the heat resistance of the cured product when combined with stereoisomers represented by Formula (2) or a mixture of stereoisomers of a compound represented by Formula (1) of the present invention. The curable composition of the present invention may comprise one or two or more of the acid anhydride-based compounds described above.

From the viewpoint of heat resistance of the cured product, the amount of the curing agent in the curable composition of the present invention when the curable composition does not comprise other epoxy compounds than the compound represented by Formula (1) above described later, based on one epoxy equivalent of stereoisomers of Formula (2) or a mixture of stereoisomers of a compound of Formula (1) of the present invention contained in the curable composition, is preferably from 0.5 to 1.5 equivalents (acid anhydride equivalent), more preferably from 0.6 to 1.2 equivalents, and still more preferably from 0.8 to 1.2 equivalents. The amount of the curing agent in the curable composition of the present invention when the curable composition comprises other epoxy compounds than the compound represented by Formula (1) above, based on one epoxy equivalent of a mixture of stereoisomers of Formula (2) or a mixture of stereoisomers of a compound of Formula (1) of the present invention, and epoxy compounds comprising other epoxy compounds than the compound represented by Formula (1) above contained in the curable composition, is preferably from 0.5 to 1.5 equivalents (acid anhydride equivalent), more preferably from 0.6 to 1.2 equivalents, and still more preferably from 0.8 to 1.2 equivalents. The mixing ratio of the curing agent in the curable composition, relative to 100 parts by mass of the curable composition in total, is preferably in the range of 10 to 80 parts by mass, and more preferably in the range of 20 to 60 parts by mass.

Curing Agents Other Than Acid Anhydride-based Compounds

Curing agents that can be contained in the curable composition of the present invention other than acid anhydride-based compounds include, for example, anion-based compounds such as amine-based compounds, phenol-based compounds, and latent curing agents.

Examples of the amine type compound include polyoxyethy lenediamine, polyoxypropylenediamine, polyoxybutylenediamine, polyoxyethylenetriamine, polyoxybutylenetriamine, polyoxypentyl enetriamine, diethylenetetramine, tetraethylenepentamine, m-xyl enediamine, trimethylhexamethylenediamine, 2-methylpentameth ylenediamine, diethylaminopropylamine, isophoronediamine, 1,3-b isaminomethylcyclohexane, bis(4-aminocyclohexyl)methane, norb ornanediamine,1,2-diaminocyclohexane, diaminodiphenylmethane, metaphenylenediamine, diaminodiphenylsulfone, N-aminoethylpip erazine, and the like.

As the phenolic type compound, they are xylylene skeleton containing phenol novolac resin, a dicyclopentadiene skeleton containing phenol novolac resin, a biphenyl skeleton containing phenol novolac resin, a terpene skeleton containing phenol novolac resin, a bisphenol A novolac, a bisphenol S novolac, a bisphenol AP novolac, a bisphenol E novolac, a bisphenol Z novolac, a tetramethyl bisphenol A novolac, a tetramethyl bisphenol F novolac, a dimethyl bisphenol F novolac,tetramethyl bisphenol S novolac, dimethyl bisphenol S novolac, tetramethyl-4,4'-biphenol novolac, tris hydroxyphenyl methane novolac, Examples thereof include resorcinol novolac, hydroquinone novolac, pyrogallol

novolac, diisopropylidene novolac, 1,1-di-4-hydroxyphenylfluorene novolac, phenolated polybutadiene novolac, phenol novolac, cresol novolac, ethylphenol novolac, butyl phenol novolac, octylphenol novolac, naphthol novolac, and the like.

Examples of the latent curing agents include dicyandiamide, adipic acid dihydrazide, sebacic acid dihydrazide, dodecanedioic acid dihydrazide, isophthalic acid dihydrazide, ketimine, imidazole compounds, dihydrazide compounds, amine adduct-based latent curing agents.

The curable composition of the present invention may comprise one or two or more of the curing agents described above.

From the viewpoint of heat resistance of the cured product, the mixing ratio of the curing agent in the curable composition, relative to 100 parts by mass of the curable composition in total, is preferably in the range of 10 to 80 parts by mass, and more preferably in the range of 20 to 60 parts by mass.

Thermal Cationic Polymerization Initiator

In one embodiment of the present invention, cationic polymerization initiators that can be contained in the curable composition include thermal cationic polymerization initiators (initiators that can produce cationic active species by means of thermal energy) and photo-cationic polymerization initiators (initiators that can produce cationic active species by means of radiation of light or electron beam). Stereoisomers of Formula (2) or a mixture or stereoisomers of a compound of Formula (1) and a thermal cationic polymerization initiator can be combined to further improve the heat resistance of the cured product.

Examples of the thermal cationic polymerization initiator include (i) aromatic sulfonium salt-based thermal cationic polymerization initiators; (ii) phosphonium salt-based thermal cationic polymerization initiators; (iii) quaternary ammonium salt-based thermal cationic polymerization initiators; (iv) aluminum complex-based thermal cationic polymerization initiators; (v) aromatic iodonium salt-based thermal cationic polymerization initiators; (vi) aromatic diazonium salt-based thermal cationic polymerization initiators, and (vii) pyridinium-based thermal cationic polymerization initiators.

(i) Examples of the thermal cationic polymerization initiators of an aromatic sulfonium salt system include (2-ethoxy-1-methyl-2-oxoethyl)methyl-2-naphthalenylsulfoniumhexafluoro antimonate, 4-(methoxycarbonyloxy)phenylbenzylmethyl sulfonium hexafluoroantimonate, 4-acetoxyphenyldimethylsulfoni umhexafluoroantimonate, 4-hydroxyphenyl(o-methylbenzyl )methylsulfoniumhexafluoroantimonate,4-hydroxyphenyl(a-naphthylmethyl)methylsulfoniumhexafluoroantimonate,diphenyl-4-(phenylthio) phenylsulfonium hexafluoroantimonate, triphenylsulfonium hexafluoroantimonate, Hexafluoroantimonate salts such as bis[4-(4-(2-hydroxyethoxy)phenylsulfonio)phenyl]su lfidobishexafluoroantimonate, bis[4-(diphenylsulfonio)phenyl ]sulfidobishexafluoroantimonate, (2-ethoxy-1-methyl-2-oxoethyl) methyl-2-naphthalenylsulfoniumhexafluorophosphate, 4-acetoxyp henylbenzylmethylsulfoniumhexafluorophosphate,4-hydroxypheny l(o-methylbenzyl)methylsulfoniumhexafluorophosphate,4-hydrox yphenyl (a-naphthylmethyl) methylsulfonium hexafluoro phosphate, diphenyl-4-(phenylthio) phenylsulfonium hexafluoro phosphate, Hexafluorophosphate salts such as bis[4-(4-(2-hydroxyethoxy)phenylsulfonio)phenyl]sulfidobishexafluoroph osphate, bis[4-(diphenylsulfonio)phenyl]sulfidobishexafluor ophosphate, 4-hydroxyphenyl(o-methylbenzyl)methylsul foniumhexafluoroarsenate, and the like, (2-ethoxy-1-methyl-2-oxoethyl)methyl-2-naphthalenylsulfoniumtetrafluoroborate,4-hydroxyphenyl(o-methylbenzyl)methylsulfonium tetrafluoroborate, 4-hydroxyphenylbenzylmethylsulfonium tetrafluoroborate, diphenyl-4-(phenylthio)phenylsulfonium tetrafluoroborate, Tetrafluoroborate salts such as triphenylsulfonium tetrafluoroborate, bis[4-(4-(2-hydroxyethoxy))phenylsulfoni o)phenyl]sulfide bistetrafluoroborate, 4-hydroxyphenyl(o-me thylbenzyl)methylsulfonium trifluoromethanesulfonate, trifluorom ethanesulfonate, and the like,a trifluoromethanesulfonate such as diphenyl-4-(phenylthio)phenylsulfonium trifluoromethanesulf onate, a 4-hydroxyphenyl(a-naphthylmethyl)methylsulfonium bis(trifluoromethanesulfone)imide, bis(trifluoromethanesulfone)im ide salts such as 4-hydroxyphenylbenzylmethylsulfonium bis(triflu oromethanesulfone)imide, (2-ethoxy-1-methyl-2-oxoethyl)methyl-2-naphthalenylsulfoniumtetrakis(pentafluorophenyl)borate, 4-(methoxycarbonyloxy)phenylbenzylmethylsulfoniumtetrakis(pe ntafluorophenyl)borate,4-hydroxyphenyl (a-naphthylmethyl) methylsulfonium tetrakis (pentafluorophenyl) borate, 4-hydroxyp henylbenzylmethylsulfonium tetrakis (pentafluorophenyl) borate, Examples thereof include tetrakis (pentafluorophenyl) borates such as triphenylsulfonium tetrakis (pentafluorophenyl) borate, bis[4-(di(4-(2-hydroxyethoxy))phenylsulfonio)phenyl]sulfidotetrakis(pe ntafluorophenyl)borate, and bis[4-(diphenylsulfonio)phenyl ]sulfidotetrakis(pentafluorophenyl)borate.

(ii) Examples of the thermal cationic polymerization initiat or of the phosphonium type salt include ethyltriphenylphosphoniu m hexafluoroantimonate and tetrabutylphosphonium hexafluoroan timonate.

(iii) Examples of the thermal cationic polymerization initiators of the quaternary ammonium salt type include a N,N-dimethyl-N-benzylanilinium hexafluoroantimonate, a diethyl-N-benzylanilinium tetrafluoroborate, a N,N-dimethyl-N-benzy lpyridinium hexafluoroantimonate, a N,N-dimethyl-N-(4-me thoxybenzyl)pyridinium hexafluoroantimonate,and N,N-diethyl-N-(4-methoxybenzyl)toluidinium hexafluoroantimonate, dimethyl-N-(4-methoxybenzyl)toluidinium hexafluoroantimonate, and the like.

(iv) Examples of the thermal cationic polymerization initiat ors of aluminum complexes include aluminum carboxylate, alumin um alkoxide, aluminum chloride, aluminum (alkoxide) acetoacetat e chelate, acetoacetanaluminium, ethylacetoacetatoaluminium, et c.

(v) Examples of the thermal cationic polymerization initiators of an aromatic iodonium salt system include phenyliodonium hexafluoroantimonate, diphenyliodonium tetrafluoroborate, diphenyliodonium tetrakis (pentafluorophenyl) borate, diphenyliodonium hexafluorophosphate, diphenyliodonium trifluoromethanesulfonate, bis (dodecylphenyl) iodonium hexafluorophosphate, bis (dodecylphenyl) iodonium tetrafluor oborate,bis(dodecylphenyl)iodonium tetrakis(pentafluoro phenyl) borate, 4-methylphenyl-4-(1-methylethyl)phenyliodonium hexafluorophosphate, 4-methylphenyl-4-(1-methylethyl)phenylio donium hexafluoroantimonate, 4-methylphenyl-4-(1-methylethy l)phenyliodonium tetrafluoroborate, 4-methylphenyl-4-(1-methylethyl)phenyliodonium tetra kis(pentafluorophenyl)borate, and the like.

(vi) Examples of the thermal cationic polymerization initiat or of the aromatic diazonium salt system include phenyldiazonium hexafluorophosphate, phenyldiazonium hexafluoroantimonate, ph enyldiazonium tetrafluoroborate and phenyldiazonium tetrakis (pe ntafluorophenyl) borate, and the like.

(vii) Examples of the pyridinium-based thermal cationic polymerization initiators include 1-benzilic-2-cyanopyridinium hexafluorophosphate, 1-benzilic-2-cyanopyridinium hexafluoro antimonate, 1-benzilic-2-cyanopyridinium tetrafluoroborate, 1-(naphthylmethyl)-2-cyanopyridinium hexafluorophosphate, 1-(naphthylmethyl)-2-cyanopyridinium hexafluoroantimonate,1-(naphthylmethyl)-2-cyanopyridiniumtetrafluoroborate, 1-(naphthy lmethyl)-2-cyanopyridiniumtetrakis(pentafluorophenyl)borate, and the like.

The thermal cationic polymerization initiators may be used alone or in combination of two or more of them.

The amount of the thermal cationic polymerization initiator in the curable composition of the present invention when the curable composition does not comprise any of other epoxy compounds than the compound represented by Formula (1) above described later, oxetane compounds described later, and vinyl ether described later, based on 100 parts by mass of stereoisomers of Formula (2) or a mixture of stereoisomers of a compound of Formula (1) of the present invention contained in the curable composition, is preferably from 0.1 to 15 parts by mass, and more preferably from 0.3 to 7 parts by mass. The amount of the thermal cationic polymerization initiator in the curable composition of the present invention when the curable composition comprises one or two or more selected from the group consisting of other epoxy compounds than the compound represented by Formula (1) above, oxetane compounds, and vinyl ether, based on 100 parts by mass of the total of stereoisomers of Formula (2) or a mixture of stereoisomers of a compound of Formula (1) of the present invention, oxetane compounds, and vinyl ether contained in the curable composition, is preferably from 0.1 to 15 parts by mass, and more preferably from 0.3 to 7 parts by mass. When the amount of the thermal cationic polymerization initiator is within the numerical range, the heat resistance of the cured product can be further improved.

More preferably, the thermal cationic polymerization initiator contained in the curable composition of the present invention is selected from the group consisting of aromatic sulfonium salt-based thermal cationic polymerization initiators, aromatic iodonium salt-based thermal cationic polymerization initiators, and aluminum complex-based thermal cationic polymerization initiators. Still more preferably, the thermal cationic polymerization initiator contained in the curable composition of the present invention is an aromatic sulfonium salt-based thermal cationic polymerization initiator.

Photo-cationic Polymerization Initiator

In one embodiment of the present invention, examples of the photo-cationic polymerization initiator contained in the curable composition of the present invention include those that produce cationic species or Lewis acid by means of radiation of an active energy ray such as visible light ray, ultraviolet ray, X-ray, or electron beam, and initiate the polymerization reaction of the cationic polymerizable compound. Examples of the photo-cationic polymerization initiator contained in the curable composition of the present invention that can be used include onium salts, metallocene complexes, iron-allene complexes, and other compounds. Examples of the onium salts that can be used include aromatic sulfonium salts, aromatic iodonium salts, aromatic diazonium salts, aromatic phosphonium salts, and aromatic selenium salts. Examples of counter ions thereof that can be used include anions such as $CF_3SO_3^-$, $BF_4^-$, $PF_6^-$, $AsF_6^-$, and $SbF_6^-$. Among them, aromatic sulfonium salt-based photo-cationic polymerization initiators are more preferably used because they have ultraviolet ray absorbing properties even in the wavelength region of 300 nm or more, and excellent curability, and thus can provide cured products having good mechanical strength and adhesive strength. The curable composition of the present invention may comprise two or more photo-cationic polymerization initiators.

Examples of the aromatic sulfonium salts include diphenyl-4 -(phenylthio)phenylsulfonium hexafluoro phosphate, 4,4'-bis(diphe nylsulfonio)diphenylsulfide bishexafluorophosphate, 4,4'-bis[di(β-hydroxyethoxy)phenylsulfonio]diphenylsulfide bishexafluorophosp hate,7-[di(p-toluyl)sulfonio]-2-isopropylthioxanthone hexafluoroa ntimonate, 7-[di(p-toluyl)sulfonio]-2-isopropylthioxanthone tetrak is(pentafluorophenyl)borate, 4-phenylcarbonyl-4'-diphenylsulfonio -diphenylsulfide hexafluoro phosphate, 4-(p-tert-butylphenylcarbo nyl)-4'-diphenylsulfidohexafluoroantimonate, 4-(p-tert-butylpheny lcarbonyl)-4'-di(p-toluyl)sulfonio-diphenylsulfidotetrakis(pentafluo rophenyl)borate, diphenyl-4-(phenylthio)phenylsulfonium hexafluo roantimonate, triphenylsulfonium trifluoromethanesulfonate, bis[4 -(diphenylsulfonio)phenyl]sulfidobishexafluoroantimonate,(4-meth oxyphenyl)diphenylsulfonium hexafluoroantimonate and the like.

Examples of the aromatic iodonium salts include diphenylio donium tetrakis(pentafluorophenyl)borate, diphenyliodonium hexa fluorophosphate, diphenyliodonium hexafluoroantimonate, di(4-no nylphenyl)iodonium hexafluorophosphate, (4-methoxyphenyl)phen yliodonium hexafluoroantimonate, and bis(4-t-butylphenyl)iodoniu m hexafluorophosphate and the like.

Examples of the aromatic diazonium salts include benzenedi azonium hexafluoroantimonate, benzenediazonium hexafluorophos phate, benzenediazonium tetrafluoroborate, and 4-chlorobenzened iazonium hexafluorophosphate.

Examples of the aromatic phosphonium salts include benzyltriphenylphosphoniumhexafluoroantimonate and the like.

Examples of the aromatic selenium salts include triphenylse lenium hexafluorophosphate and the like.

Examples of the iron-allene complexes include xylen-cyclop entadienyl iron (II) hexafluoroantimonate, cumene-cyclopentadien yl iron (II) hexafluorophosphate, and xylene-cyclopentadienyl iron (II) tris (trifluoromethylsulfonyl) methanide.

The amount of the photo-cationic polymerization initiator in the curable composition of the present invention when the curable composition does not comprise any of other epoxy compounds than the compound represented by Formula (1) above described later, oxetane compounds described later, and vinyl ether described later, based on 100 parts by mass of stereoisomers of Formula (2) or a mixture of stereoisomers of a compound of Formula (1) of the present invention contained in the curable composition, is preferably from 0.01 to 20 parts by mass, and more preferably from 0.1 to 5 parts by mass. The amount of the photo-cationic polymerization initiator in the curable composition of the present invention when the curable composition comprises one or two or more selected from the group consisting of other epoxy compounds than the compound represented by Formula (1) above, oxetane compounds, and vinyl ether, based on 100 parts by mass of the total of stereoisomers of Formula (2) or a mixture of stereoisomers of a compound of Formula (1) of the present invention, oxetane compounds, and vinyl ether contained in the curable composition, is preferably from 0.01 to 20 parts by mass, and more preferably from 0.1 to 5 parts by mass. When the amount of the photo-cationic polymerization initiator is within the numerical range, the heat resistance of the cured product can be further improved.

Curing accelerators

According to one embodiment of the present invention, as the curing accelerator contained in the curable composition, for example, triphenylphosphine, triphenylbenzylphosphonium tetraphenylborate, tetrabutyl phosphonium diethylphosphodithio ate, tetraphenylphosphonium bromide, tetrabutylphosphonium bromide, tetra-n-butylphosphonium tetrafluoroborate, tetra-n-bu tylphosphonium tetraphenylborate, methyltriphenylphosphonium bromide, ethyltriphenylphosphonium bromide, ethyltriph enylphosphonium iodide,ethyltriphenylphosphonium acetate, methyl tri-n-butylphosphonium dimethylphosphate, n-butyltri phenylphosphonium bromide, benzyltriphenylphosphonium chloride, Phosphines such as tetraphenylphosphonium tetraphenylborate and its quaternary salts, 2-ethyl-4-methylimid azole, 1,2-dimethylimidazole, 1-benzilic-2-phenylimidazole, 2-phenylimidazole, 1-(2-cyanoethyl)-2-ethyl-4-methylimidazole, 2,4-diamino-6-[2-methylimidazolyl-(1)]ethyl-s-triazine, 2-phenyl-i midazoline,imidazoles such as 2,3-dihydro-1H-pyrrolo[1,2-a]b enzimidazole, tertiary amines such as tris(dimethylaminome thyl)phenol, tetrabutylammonium bromide and its quaternary salts, superbasic organic compounds such as 1,8-diazabicyclo(5,4 ,0)nonene-5, zinc octylate, zinc laurate, zinc stearate, Examples thereof include metal-organic chelate compounds such as tin octylate and the like, metal-organic chelate compounds such as benzoylacetone zinc chelate, dibenzoylmethane zinc chelate and ethylzinc acetoacetate chelate, and tetra-n-butylsulfonium-o,o-die thylphosphorodithionate etc. may be mentioned.

Among them, imidazole-based curing accelerators are more preferable, and 2-methyl-imidazole, 2-phenylimidazole, and 2-ethyl-4-methylimidazole are still more preferable because they can be combined with stereoisomers of Formula (2) of the present invention or a mixture of stereoisomers of a compound of Formula (1) to further improve the heat resistance of the cured product.

The curable composition of the present invention may comprise one or two or more of the curing accelerators as described above.

From the viewpoint of heat resistance of the cured product, the amount of the curing accelerator in the curable composition of the present invention when the curable composition does not comprise other epoxy compounds than the compound represented by Formula (1) above described later, based on 100 parts by mass of stereoisomers of Formula (2) or a mixture of stereoisomers of a compound of Formula (1) of the present invention contained in the curable composition, is preferably from 0.01 to 10 parts by mass, more preferably from 0.05 to 8 parts by mass, and still more preferably from 0.1 to 6 parts by mass. The amount of the curing accelerator in the curable composition of the present

invention when the curable composition comprises other epoxy compounds than the compound represented by Formula (1) above, based on 100 parts by mass in total of stereoisomers of Formula (2) or a mixture of stereoisomers of a compound of Formula (1) of the present invention, and epoxy compounds other than the compound represented by Formula (1) contained in the curable composition, is preferably from 0.01 to 10 parts by mass, more preferably from 0.05 to 8 parts by mass, and still more preferably from 0.1 to 6 parts by mass.

Other Epoxy Compounds than Compound Represented by Formula (1) Above

In one embodiment of the present invention, the curable composition may comprise other epoxy compounds than the compound represented by Formula (1) above (hereinafter may be referred to as "other epoxy compounds") depending on the application. Examples of the other epoxy compounds include glycidyl ether epoxides, glycidyl ester epoxides, glycidyl amine epoxides, alicyclic epoxides, and other epoxides, and oligomers and polymers thereof, and tetraphenylethane epoxy resins. The polymers may be epoxy resins obtained by polymerization of glycidyl ether epoxides, glycidyl ester epoxides, glycidyl amine epoxides, alicyclic epoxides, and other epoxides, and are preferably glycidyl ether epoxy resins such as bisphenol A, bisphenol F, bisphenol AD, and bisphenol S, and more preferably bisphenol A epoxy resins.

As glycidyl ether type epoxide, For example, bisphenol A diglycidyl ether, bisphenol F diglycidyl ether, bisphenol S diglycidyl ether, tetramethylbiphenol diglycidyl ether, hydrogenated bisphenol A diglycidyl ether, glycidyl ether of dihydric phenol such as brominated bisphenol A diglycidyl ether, dihydroxynaphthyl cresol triglycidyl ether, tris (hydroxyphenyl) methane triglycidyl ether, dinaphthyltriol triglycidyl ether, phenol novolac glycidyl ether, cresol novolac glycidyl ether,xylylene skeleton-containing phenol novolac glycidyl ether, dicyclopentadiene skeleton-containing phenol novolac glycidyl ether, Biphenyl skeleton-containing phenol novolac glycidyl ether, terpene novolac glycidyl ether, bisphenol A novolac glycidyl ether, bisphenol S novolac glycidyl ether, bisphenol AP novolac glycidyl ether, bisphenol E novolac glycidyl ether, bisphenol Z novolac glycidyl ether, tetramethyl bisphenol A novolac glycidyl ether, tetramethyl bisphenol F novolac glycidyl ether,dimethyl bisphenol F novolac glycidyl ether, tetramethyl bisphenol S novolac glycidyl ether, Dimethylbisphenol S novolac glycidyl ether, tetramethyl-4,4'-bip henol novolac glycidyl ether, tris-hydroxyphenyl methane novolac glycidyl ether, resorcinol novolac glycidyl ether, hydroquinone volac glycidyl ether, diisopropylidene novolac glycidyl ether, 1,1-di-4-hydroxyphenyl fluorene novolac glycidyl ether, phenolated polybutadiene novolac glycidyl ether, ethylphenol novolac glycidyl ether, butylphenol novolac glycidyl ether,octylphenol novolac glycidyl ether, naphthol novolac glycidyl ether, glycidyl ether of polyhydric phenol such as hydrogenated phenol novolac glycidyl ether, ethylene glycol diglycidyl ether, Propylene glycol diglycidyl ether, tetramethylene glycol diglycidyl ether, 1,6-hexanediol diglycidyl ether, cyclohexanedimethylol diglycidyl ether, and polyethylene glycol diglycidyl ether, Examples thereof include glycidyl ethers of dihydric alcohols such as polypropylene glycol diglycidyl ether, trimethylolpropane triglycidyl ether, glycerin triglycidyl ether, glycidyl ether of polyhydric alcohols such as pentaerythritol tetraglycidyl ether, sorbitol hexaglycidyl ether, and polyglycerin polyglycidyl ether, and triglycidyl isocyanurate etc. may be mentioned..

As the glycidyl ester type epoxide, for example glycidyl esters of carboxylic acids such as glycidyl methacrylate, diglycidyl ester phthalate, diglycidyl ester isophthalate, diglycidyl ester terephthalate, diglycidyl ester cyclohexanedicarboxylate, and triglycidyl ester trimet acid, and polyepoxides of glycidyl ester type etc. may be mentioned.

As a glycidylamine type epoxide, for example N,N-diglyc idylaniline, N,N-diglycidyl toluidine, N,N,N', N'-tetraglycidyldiam inodiphenylmethane, N,N,N', N'-tetraglycidyldiaminodipheny lsulfone, N,N,N', and N'-tetraglycidyldiethyldiphenylmethane, bis (N,N-diglycidylaminocyclohexyl) methane (N,N,N', hydride of N'-tetraglycidyldiaminodiphenylmethane), N,N,N', N'-tetraglycidyl-1,3 -(bisaminomethyl)cyclohexane (N,N,N', hydrogen product of N'-tetraglycidylxylyldiamine),and glycidyl heterocyclic amines such as triglycidyl-p-aminophenol, N-glycidyl-4-glycidyloxypyrrolidone, etc. may be mentioned.

As alicyclic epoxides, for example vinylcyclohexene dioxide, limonene dioxide, dicyclopentadiene dioxide, bis (2,3-epoxy cyclopentyl) ether, ethylene glycol bisepoxydicyclopentyl ether, 3,4-epoxy-6-methylcyclohexylmethyl 3', 4'-epoxycyclohexanec arboxylate,3,4-epoxy-1-methylcyclohexyl 3,4-epoxy-1-methylhe xanecarboxylate, 3,4-epoxy-3-methylhexanecarboxylate, 3,4-epoxy-5-methylcyclohexyl methyl 3,4-epoxy-5-methylcy clohexanecarboxylate,2-(3,4-epoxycyclohexyl-5,5-spiro-3,4-epo xy)cyclohexane-metadioxane, methylenebis(3,4-epoxycyclohex ane), (3,3 ', 4,4 '-diepoxy)bicyclohexyl, 1,2-epoxy-(2-oxylany l)cyclohexane adducts of 2,2-bis(hydroxymethyl)-1-butanol, and tetrahydroindene diepoxide etc. may be mentioned. The curable composition of the present invention may comprise one or two or more of the epoxy compounds other than the compound represented by Formula (1) above as described above.

From the viewpoint of heat resistance of the cured product, the amount of epoxy compounds other than the compound represented by Formula (1) above as described above, relative to 100 parts by mass of the curable composition in total, is preferably in the range of 1 to 90 parts by mass, and more preferably in the range of 5 to 85 parts by mass.

In preferred embodiments of the present invention, epoxy compounds other than the compound represented by Formula (1) above contained in the curable composition of the present invention are selected from the group consisting of glycidyl ether epoxides, glycidyl ester epoxides, alicyclic epoxides, tetraphenylethane epoxy resins, and glycidyl ether

epoxy resins.

Reactive Diluent

In one embodiment of the present invention, the curable composition may further comprise a reactive diluent for having lower viscosity. Examples of the reactive diluent include butylglycidyl ethers, 2-ethylhexylglycidyl ethers, glycidyl ethers of C12-13 mixed alcohols, and 1,2-epoxy-4-vinylcyclohexane. The curable composition may comprise one or two or more of the reactive diluents as described above. The mixing ratio of the reactive diluent may be appropriately adjusted so that the curable composition containing the reactive diluent has a desired viscosity.

Oxetane Compound

In one embodiment of the present invention, the curable composition may comprise an oxetane compound. Examples of oxetane compounds include 1,4-bis[(3-ethyl-3-oxetanylmet hoxy)methyl]benzene, 3-ethyl-3-(phenoxymethyl)oxetane, di[(3-ethyl-3-oxetanyl)methyl] ether, 3-ethyl-3-(cyclohexyloxymethyl )oxetane, phenol novolac oxetane,1,3-bis[(3-ethyloxetan-3-yl)]methoxybenzene, oxetanyl silicicate, bis[1-ethyl(3-oxetan yl)]methyl ether, 4,4'-bis(3-ethyl-3-oxetanylmethoxy)biphenyl, ethylene glycol (3-ethyl-3-oxetanylmethyl)ether, diethylene glycolbis(3-ethyl-3-oxetanylmethyl)ether, bis(3-ethyl-3-oxetan ylmethyl)diphenoate, trimethylolpropane tris(3-ethyl-3-oxeta nylmethyl)ether, pentaerythritol tetrakis(3-ethyl-3-oxetanylmet hyl)ether, and phenol novolac type oxetane. The curable composition of the present invention may comprise one or two or more of the oxetane compounds as described above.

From the viewpoint of heat resistance of the cured product, the amount of the oxetane compound in the curable composition of the present invention, relative to 100 parts by mass of the curable composition in total, is preferably in the range of 1 to 90 parts by mass, and more preferably in the range of 5 to 85 parts by mass.

Vinyl Ether Compound

According to one embodiment of the present invention, the curable composition may contain a vinyl Ether compound. Examples of the vinyl ether compounds include monofunctional vinyl ether, such as methyl vinyl ether, ethyl vinyl ether, and butyl vinyl ether, for example, Butanediol divinyl ether, cyclohexanedimethanol divinyl ether, cyclohexanediol divinyl ether, trimethylolpropanetr ivinyl ether, pentaerythritol tetravinyl ether, glycerol trivinyl ether, triethylene glycol divinyl ether, and diethylene glycol divinyl ether, hydroxyethylvinyl ether, cyclohexanedimethanol monovinyl ether, cyclohexanediol monovinyl ether,9-hydroxynonyl vinyl ether, propylene glycol monovinyl ether, neopentyl glycol monovinyl ether, Glycerol divinyl ether, trimethylolpropanedivinyl ether, trimethylolpropanemonovinyl ether, pentaerythritol divinyl ether, pentaerythritol trivinyl ether, diethylene glycol monovinyl ether, tetraethylene glycol monovinyl ether, tricyclodecanediol monovinyl ether, vinyl ether compounds having hydroxyl groups such as tricyclodecanedimethanol monovinyl ether and acrylic acid 2-(2-vinyloxyethoxy)ethyl,Examples thereof include vinyl ethers having different functional groups such as 2-(2-vinyloxyethoxy)ethyl methacrylate. The curable composition of the present invention may comprise one or two or more of the vinyl ether compounds as described above.

From the viewpoint of heat resistance of the cured product, the amount of the vinyl ether compound in the curable composition of the present invention, relative to 100 parts by mass of the curable composition in total, is preferably in the range of 1 to 90 parts by mass, and more preferably in the range of 5 to 85 parts by mass.

Compound Having Hydroxy Group

In one embodiment of the present invention, the curable composition may further comprise a compound having a hydroxy group. Inclusion of a compound having a hydroxy group in the curable composition enables the curing reaction to be progressed slowly. Examples of the compound having a hydroxy group include ethylene glycol, diethylene glycol, and glycerol. The curable composition of the present invention may comprise one or two or more of the compounds having a hydroxy group as described above.

From the viewpoint of heat resistance of the cured product, the amount of the compound having a hydroxy group in the curable composition of the present invention, relative to 100 parts by mass of the curable composition in total, is preferably in the range of 0.1 to 10 parts by mass, and more preferably in the range of 0.2 to 8 parts by mass.

Solvent / Others

In one embodiment of the present invention, the curable composition may further comprise a solvent. Examples of the solvent include methyl ethyl ketone, ethyl acetate, toluene, methanol, and ethanol.

In one embodiment of the present invention, the curable composition may comprise various additives without

impairing the characteristics. Examples of the additives include fillers, silane coupling agents, mold release agents, coloring agents, flame retardants, antioxidants, light stabilizers, and plasticizers, antifoamers, light stabilizers, coloring agents such as pigments and dyes, plasticizers, pH adjusters, anti-coloring agents, matting agents, deodorants, weathering agents, antistatic agents, yarn abrasion reducing agents, slipping agents, and ion exchangers.

Production of Curable Composition

In one embodiment of the present invention, components to be further contained in the curable composition and a method of preparing the curable composition in production of the curable composition can be appropriately selected according to the common general technical knowledge widely known by those skilled in the art.

In preferred embodiments of the present invention, the method of preparing the curable composition enables production of the curable composition, for example, by kneading or mixing the stereoisomers of Formula (2) or the mixture of stereoisomers of a compound of Formula (1) of the present invention as described above, and optionally the components as described above, and other components added as appropriate.

The kneading or mixing method is not particularly limited, and mixing can be done, for example, using a mixing or kneading machine such as a planetary mixer, a twin screw extruder, heated rolls, or a kneader.

Cured Product and Production Method Thereof

In one embodiment of the present invention, the cured product is obtained by curing the curable composition of the present invention described above. The method of curing the curable composition is not particularly limited, and can be performed by heating or photoirradiation as appropriate.

When the curable composition is cured by heating, the curable composition is preferably heated in multiple steps, taking into account the high reactivity of the epoxy compound. This allows the curing reaction to proceed sufficiently. For example, the curing reaction can be carried out through first heating at 90 to 110°C for 10 to 150 minutes and second heating at 140 to 160°C for 10 to 400 minutes. For example, the curing reaction can be carried out through first heating at 100 to 130°C for 10 to 150 minutes, second heating at 140 to 160°C for 10 to 150 minutes, third heating at 170 to 200°C for 60 to 180 minutes, and fourth heating at 210 to 250°C for 10 to 150 minutes. For example, the curing reaction can be carried out through first heating at 100 to 130°C for 10 to 150 minutes, second heating at 140 to 200°C for 10 to 150 minutes, and third heating at 210 to 250°C for 10 to 150 minutes. For example, the curing reaction can be carried out through first heating at 80 to 100°C for 10 to 150 minutes, second heating at 110 to 120°C for 10 to 150 minutes, third heating at 130 to 140°C for 60 to 180 minutes, fourth heating at 150 to 170°C for 10 to 150 minutes, fifth heating at 180 to 200°C for 60 to 180 minutes, and sixth heating at 210 to 230°C for 60 to 240 minutes. For example, the curing reaction can be carried out through first heating at 100 to 110°C for 10 to 150 minutes, second heating at 120 to 150°C for 10 to 150 minutes, third heating at 160 to 220°C for 10 to 150 minutes, and fourth heating at 230 to 250°C for 10 to 150 minutes. However, the curing reaction is not limited to them, and is preferably carried out with appropriate changes, taking into account the amount of the epoxy compound, and the characteristics of the other compounds contained in the curable composition and the like.

When the curable composition is cured by irradiating active energy ray such as visible light ray, ultraviolet ray, X-ray, or electron beam, the type of the active energy ray used and the conditions are preferably changed as appropriate depending on the composition of the curable composition. In one embodiment, ultraviolet ray is preferably irradiated so that the accumulated amount of light represented by the product of the radiation intensity and the radiation time period is from 10 to 5,000 $mJ/cm^2$. When the accumulated amount of light to the curable composition is within the numerical range described above, active species derived from the photo-cationic polymerization initiator can be produced sufficiently. The productivity can also be improved.

Properties of Cured Product

In one embodiment of the present invention, the heat resistance of the cured product can be evaluated by measuring the glass transition temperature. The glass transition temperature is preferably higher from the viewpoint of imparting heat resistance.

The glass transition temperature can be measured by differential scanning calorimetry (DSC).

Measurement of the glass transition temperature by DSC can be easily performed by using a commercially available differential scanning calorimeter (for example, product name: DSC7020, manufactured by Hitachi High-Tech Science Corporation).

The glass transition temperature of the cured product of the present invention by DSC may be 140°C or higher, and is preferably 150°C or higher, and more preferably 170°C or higher. The upper limit is not particularly limited, and is preferably 400°C or lower.

In one embodiment of the present invention, the dielectric properties of the cured product can be evaluated by measuring at least one selected from dielectric constant and dielectric dissipation factor (tanδ). The dielectric constant and dielectric dissipation factor are preferably lower from the viewpoint of reducing energy loss.

Measurement of the dielectric constant and the dielectric dissipation factor of the cured product can be measured by a cavity resonator perturbation method.

Measurement of the dielectric constant and the dielectric dissipation factor can be easily performed by using a commercially available measurement resonator (for example, ADMS01Nc1, manufactured by AET, Inc.) and analyzer (for example, vector network analyzer MS46122B, manufactured by Anritsu Corporation).

The dielectric constant at 10 GHz of the cured product of the present invention is preferably from 2.50 to 2.72.

The dielectric dissipation factor at 10 GHz of the cured product of the present invention may be 0.0150 or less, preferably 0.0140 or less, and more preferably 0.0120 or less. The lower limit is not particularly limited, and is preferably 0.0050 or more.

In preferred embodiments of the present invention, a curable composition comprising stereoisomers of Formula (2) or a mixture of stereoisomers of a compound of Formula (1) as described above, as compared with a curable composition that does not comprise stereoisomers of Formula (2) or a mixture of stereoisomers of a compound of Formula (1) as described above, can have improved heat resistance and dielectric properties.

Application of Cured Product

Applications of the curable composition and/or the cured product include, specifically, materials for paints for coating base materials such as metals, resin films, glass, paper, and wood; surface protective films of semiconductor devices and organic thin film devices (for example, organic electroluminescent devices and organic thin-film solar cells); hard coatings; coatings for antifouling films, antireflection films, and the like; adhesives; pressure sensitive adhesives; various optical members such as lenses, prisms, filters, image display materials, lens arrays, sealers and reflector materials for photosemiconductor devices, sealers for semiconductor devices, optical waveguides, light guiding panels, light diffuser, diffractive elements, and optical adhesives; casting materials; interlayer insulators; protective insulating films for print-oriented substrates; fiber-reinforced composite materials; and the like.

EXAMPLES

The present invention will be described in more detail with reference to Examples below, but is not limited to the Examples.

Preparation Example 1: Preparation of Epoxy Compound (A-1)

To a reactor equipped with a thermometer, a stirrer, a reflux tube, and a dropping apparatus were added 40.0 g of a diolefin compound represented by Formula (4), 40.0 g of toluene, 1.44 g of sodium tungstate dihydrate, 1.0 g of hexadecyltrimethylammonium methyl sulphate, 0.64 g of phenylphosphonate, 2.78 g of anhydrous sodium sulfate, and 39.2 g of 45% aqueous hydrogen peroxide, and then the mixture was reacted at 30°C for 11 hours.

[Chem 6]

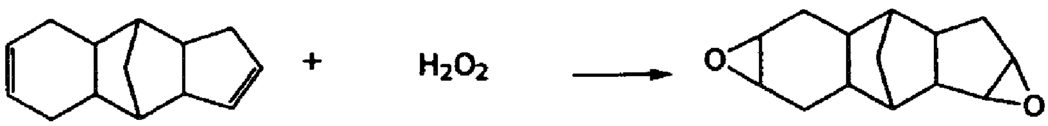

(4) (5)

After the reaction, the reaction was washed once with 15 g of a 10% aqueous sodium carbonate solution, once with 15 g of a 10% aqueous sodium sulfite solution, and three times with 20 g of a saturated aqueous sodium sulfate solution for separation. The organic layer after washing and separation was subjected to vacuum distillation (60°C, 50 hPa) with a rotary evaporator, and 53 g of the obtained crude product was added to and dissolved in 90 g of isopropanol. Thereafter, the solution was cooled to -10°C for crystallization, and filtered, followed by drying of the residue to obtain 33.3 g of white crystals (epoxy compound (A-1) represented by Formula (5)).

The obtained epoxy compound (A-1) was analyzed by gas chromatography (GC). Specifically, the analysis

was performed under the following conditions.

[Analysis Conditions]

Measurement equipment: Intuvo 9000 GC System (Agilent Technologies, Inc.)
Column: HP-5MS (Agilent Technologies, Inc.), length: 30.0 m, inner diameter: 320 μm, film thickness: 250 μm
Liquid phase: (5%-phenyl)-methylpolysiloxane
Carrier gas: $N_2$
Flow rate: 5.2 mL/min
Split ratio: 60:1
Temperature of sample inlet: 200°C
Temperature of detector: 250°C
Column temperature rising condition: 50°C (5 minutes), 50 to 150°C (5°C/min), 150 to 250°C (10°C/min), 250°C (10 minutes)

The GC analysis demonstrated that the proportion of the peak of the primary isomer in the peaks derived from the compound represented by Formula (5) (A-1) was 77%. FIG. 1 shows a gas chromatograph of the obtained epoxy compound (A-1).

Preparation Example 2: Further Purification of Epoxy Compound (A-1)

Further, the obtained epoxy compound (A-1) was repeatedly recrystallized in isopropanol to obtain a sample with a proportion of the peak of the primary isomer increased to 96%. The obtained sample was subjected to $^{1}$H- and $^{13}$C-NMR spectrum analyses, followed by two-dimensional NMR (NOESY). Specifically, the analyses were performed under the following conditions.

[Measurement Conditions for $^{1}$H-NMR]

Measurement equipment: AVANCE II 800US2 (Bruker Corporation)
Solvent: $CDCl_3$
Pulse angle: 90° pulse
Sample concentration: 10% by mass
Accumulation times: 4 times

[Measurement Conditions for $^{13}$C-NMR]

Measurement equipment: AVANCE II 800US2 (Bruker Corporation)
Solvent: $CDCl_3$
Pulse angle: 30°pulse
Sample concentration: 10% by mass
Accumulation times: 32

FIG. 2 shows the $^{1}$H-NMR spectrum of the sample obtained in Preparation Example 2, and FIG. 3 shows the $^{13}$C-NMR spectrum thereof.

($^{1}$H-NMR spectrum of the sample obtained in Preparation Example 2)

$^{1}$H NMR (800 MHz, $CDCl_3$) δ (ppm) 1.298 (d, 1H), 1.502 (m, 2H), 1.681 (d, 1H), 1.738 (m, 2H), 1.812 (m, 1H), 1.912 (q, 1H), 1.986 (m, 2H), 2.109 (m, 2H), 2.364 (m, 1H), 2.434 (m, 1H), 3.102 (m, 2H), 3.297 (d, 1H), 3.518 (t, 1H).

($^{13}C$-NMR spectrum of the sample obtained in Preparation Example 2)

$^{13}C$ NMR (800 MHz, $CDCl_3$) δ (ppm) 27.236, 27.426, 27.949, 30.289, 31.170, 37.695, 44.649, 45.103, 47.037, 47.877, 50.511, 50.632, 60.341, 61.612.

Configuration analysis of the samples was performed by two-dimensional NMR (NOESY method), determining that the structure of the peak of the primary isomer in the compound (A-1) was the structure of Formula (6) below. FIG. 4 shows a part of the NOESY spectrum of the peak component used in the configuration analysis, and FIG. 5 shows an enlarged figure thereof.

[Chem 7]

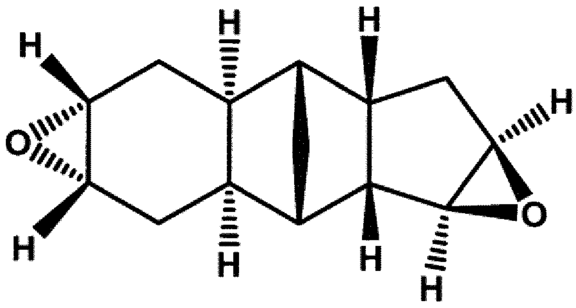

(6)

Compounds and the like used in Examples and Comparative Examples described below are shown below.

<Component (A): Epoxy Compound>

Components (A) used were as follows:

(A-1): Epoxy compound obtained by the method described in Preparation Example 1 (hereinafter also referred to as simply "epoxy compound (A-1)");
(A-2): Bisphenol A liquid epoxy resin (product name: YD-128, manufactured by Nippon Steel & Sumikin Chemical Co., Ltd.)
(A-3): Tetraphenylethane epoxy resin (product name: jER1031S, manufactured by Mitsubishi Chemical Corporation)
(A-4): Alicyclic epoxide (product name: CELLOXIDE 2021P, manufactured by Daicel Corporation)

<Component(B): Acid Anhydride-based Compound>

Components (B) used were as follows:
(B-1): A mixture of 4-methylhexahydrophthalic anhydride and hexahydrophthalic anhydride (product name: MH-700, manufactured by New Japan Chemical Co., Ltd.)

<Component (C): Curing Accelerator>

Components (C) used were as follows:
(C-1): 2-ethyl-4-methylimidazole (product name: 2E4MZ, manufactured by Shikoku Chemicals Corporation)

Examples 1 and 2, and Comparative Examples 1 to 4: Preparation of Curable Composition Containing Epoxy Compound (A-1) and Evaluation Thereof

(1) Preparation of Curable Composition and Cured Product

Example 1

Fifty parts by mass of the epoxy compound (A-1), 50 parts by mass of the epoxy compound (A-2), 0.9 equivalents of the acid anhydride-based compound (B-1) to 1 equivalent of the component (A), and 0.2 parts by mass of the curing

accelerator (C-1) were mixed to prepare a curable composition. The thus-obtained curable composition was heated in a hot air circulating oven at 100°C for 2 hours, and then at 150°C for 5 hours to obtain a cured product. Table 1 summarizes the measurement results.

Comparative Examples 1 and 2

Curable compositions and cured products thereof were prepared in the same manner as in Example 1, except that the compositions of the curable compositions were changed as shown in Table 1 below.

Example 2

A curable composition and a cured product thereof were prepared in the same manner as in Example 1, except that the composition of the curable composition was changed as shown in Table 2 below.

Comparative Examples 3 and 4

Curable compositions and cured products thereof were prepared in the same manner as in Example 1, except that the compositions of the curable compositions were changed as shown in Table 2 below.

(2) Estimation of Physical Properties

<Heat Resistance>

The glass transition temperatures of the cured products obtained in Examples 1 and 2, and Comparative Examples 1 to 4 were measured by raising the temperature from 30 to 300°C at 10 °C/min using a differential scanning calorimeter (product name: DSC7020, manufactured by Hitachi High-Tech Science Corporation), which were considered as the heat resistance of the cured products. Here, the glass transition temperature was measured based on "midpoint glass transition temperature: $T_{mg}$" described in JIS K7121, "Testing Methods for Transition Temperatures of Plastics." Tables 1 and 2 summarize the measurement results.

<Dielectric Constant / Dielectric Dissipation Factor>

The dielectric constant and dielectric dissipation factor were measured under the following conditions. Tables 1 and 2 summarize the measurement results.

- Test method: Cavity resonator perturbation method (according to the JIS C2565 standard)
- Measurement items: dielectric constant and dielectric dissipation factor
- Shape of test piece: 3 mm × 80 mm × 1 mm
- Measurement condition: frequency 10 GHz
- Measurement temperature: 23°C
- Measurement times: n = 3 (three test pieces were prepared from the same cured product, and the measurement values from the test pieces were averaged)
- Measurement equipment:

    Measuring resonator: ADMS01Nc1, manufactured by AET, Inc.

    Analyzer: a vector network analyzer MS46122B, manufactured by Anritsu Corporation

[Table 1]

| | | | Ex. 1 | Com. Ex. 1 | Com. Ex. 2 |
|---|---|---|---|---|---|
| Composition | Epoxy Compound | A-1 | 50 | | |
| | | A-2 | 50 | 100 | 50 |
| | | A-3 | | | |
| | | A-4 | | | 50 |
| | Acid Anhydride-Based Compound | B-1 | 112 | 90 | 109 |
| | Curing Accelerator | C-1 | 0.2 | 0.2 | 0.2 |
| Heat Resistance (°C) | | | 176 | 132 | 153 |
| Dielectric Constant | | | 2.72 | 2.81 | 2.81 |
| Dielectric Dissipation Factor | | | 0.0118 | 0.0152 | 0.0185 |

[Table 2]

| | | | Ex. 2 | Com. Ex. 3 | Com. Ex. 4 |
|---|---|---|---|---|---|
| Composition | Epoxy Compound | A-1 | 50 | | |
| | | A-2 | | | |
| | | A-3 | 50 | 100 | 50 |
| | | A-4 | | | 50 |
| | Acid Anhydride-Based Compound | B-1 | 114 | 84 | 107 |
| | Curing Accelerator | C-1 | 0.2 | 0.2 | 0.2 |
| Heat Resistance (°C) | | | 180 | 158 | 169 |
| Dielectric Constant | | | 2.63 | 2.93 | 2.73 |
| Dielectric Dissipation Factor | | | 0.0114 | 0.0158 | 0.0160 |

The results shown above demonstrate that the cured products from the curable compositions in embodiments of the present invention exhibit excellent heat resistance and excellent dielectric properties.

## Claims

**1.** A stereoisomer of an epoxy compound represented by Formula (1) below:

the stereoisomer being represented by Formula (2):

[Chem 1]

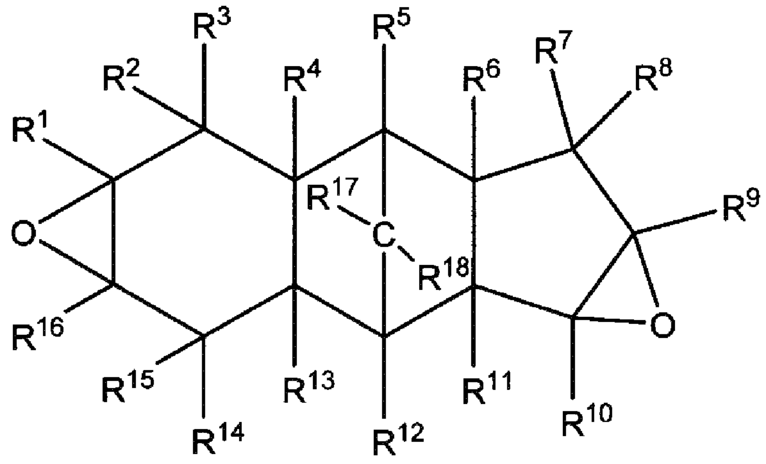

( 1 )

wherein, in Formula (1),
$R^1$ to $R^{18}$ are each independently selected from the group consisting of hydrogen, an alkyl group, and an alkoxy group;

[Chem 2]

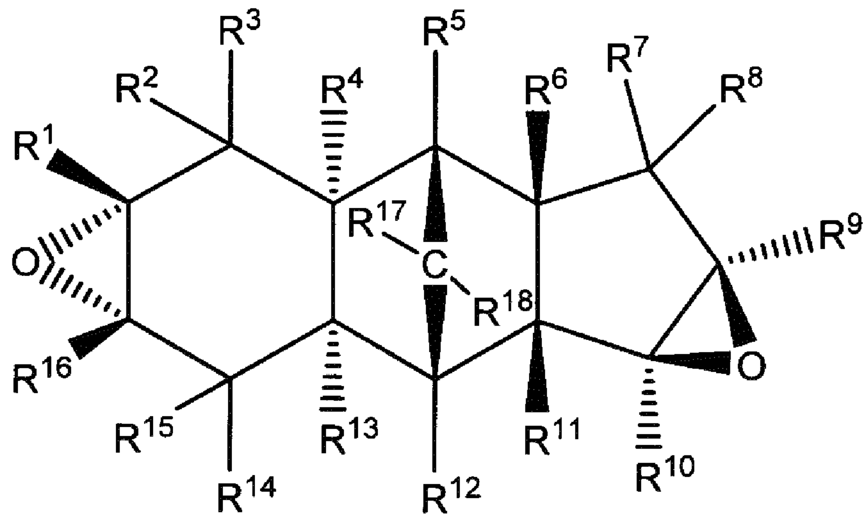

( 2 )

wherein, in Formula (2),
$R^1$ to $R^{18}$ are each the same as in Formula (1).

2. The stereoisomer according to claim 1, wherein $R^1$ to $R^{18}$ are all hydrogen.

3. A mixture of stereoisomers of a compound represented by Formula (1), wherein the amount of the stereoisomer according to claim 1 or 2 relative to the total amount of the stereoisomer mixture is 60 mol% or more.

4. A curable composition comprising the stereoisomer according to claim 1 or 2 or the mixture according to claim 3.

5. The curable composition according to claim 4, further comprising at least one selected from the group consisting of a curing agent, a thermal cationic polymerization initiator, and a photo-cationic polymerization initiator.

6. The curable composition according to claim 5, wherein the curing agent is at least one selected from an acid anhydride-based compound and an anion-based compound.

7. The curable composition according to claim 6, wherein the anion-based compound is at least one selected from the group consisting of an amine-based compound, a phenol-based compound, a thiol-based compound, and a latent curing agent.

8. The curable composition according to any one of claims 4 to 7, further comprising a curing accelerator.

9. The curable composition according to claim 8, wherein the curing accelerator is an imidazole-based curing accelerator.

10. The curable composition according to any one of claims 4 to 9, further comprising at least one selected from the group consisting of an epoxy compound other than the compound represented by Formula (1), an oxetane compound, and vinyl ether.

11. A cured product obtained by curing the curable composition according to any one of claims 4 to 10.

12. A method of producing a cured product, comprising a step of curing the curable composition according to any one of claims 4 to 10.

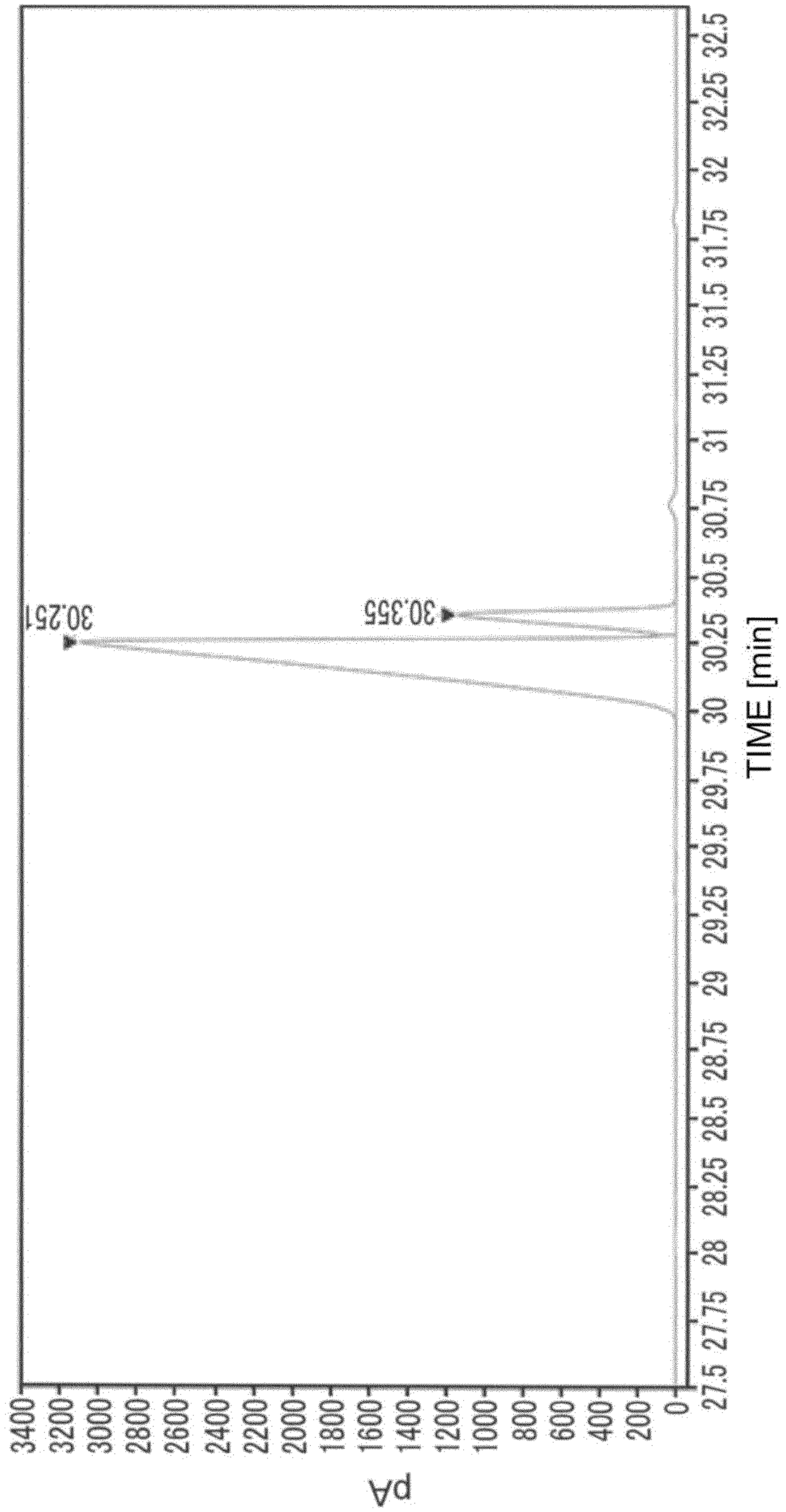


FIG. 1

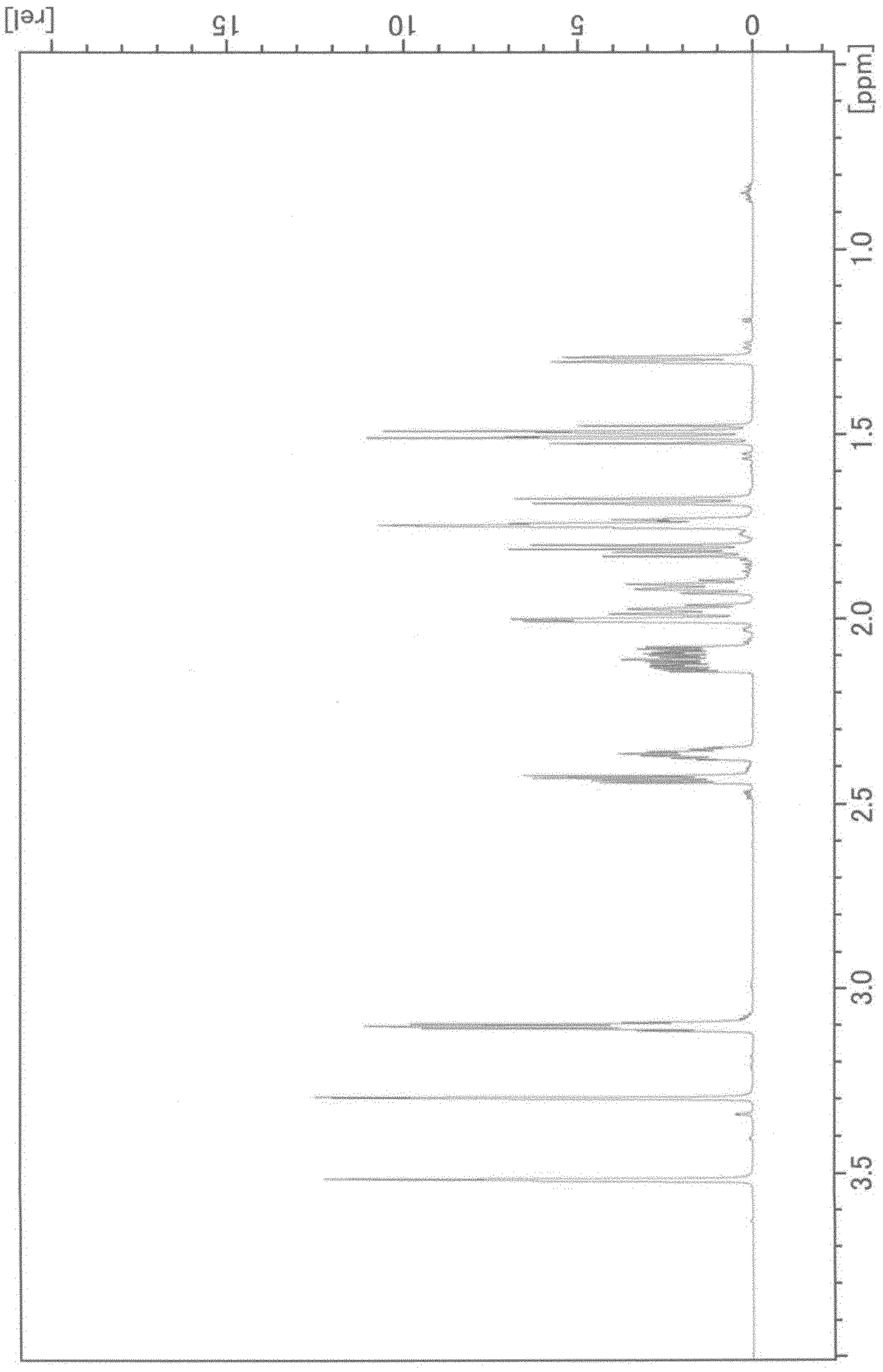


FIG. 2

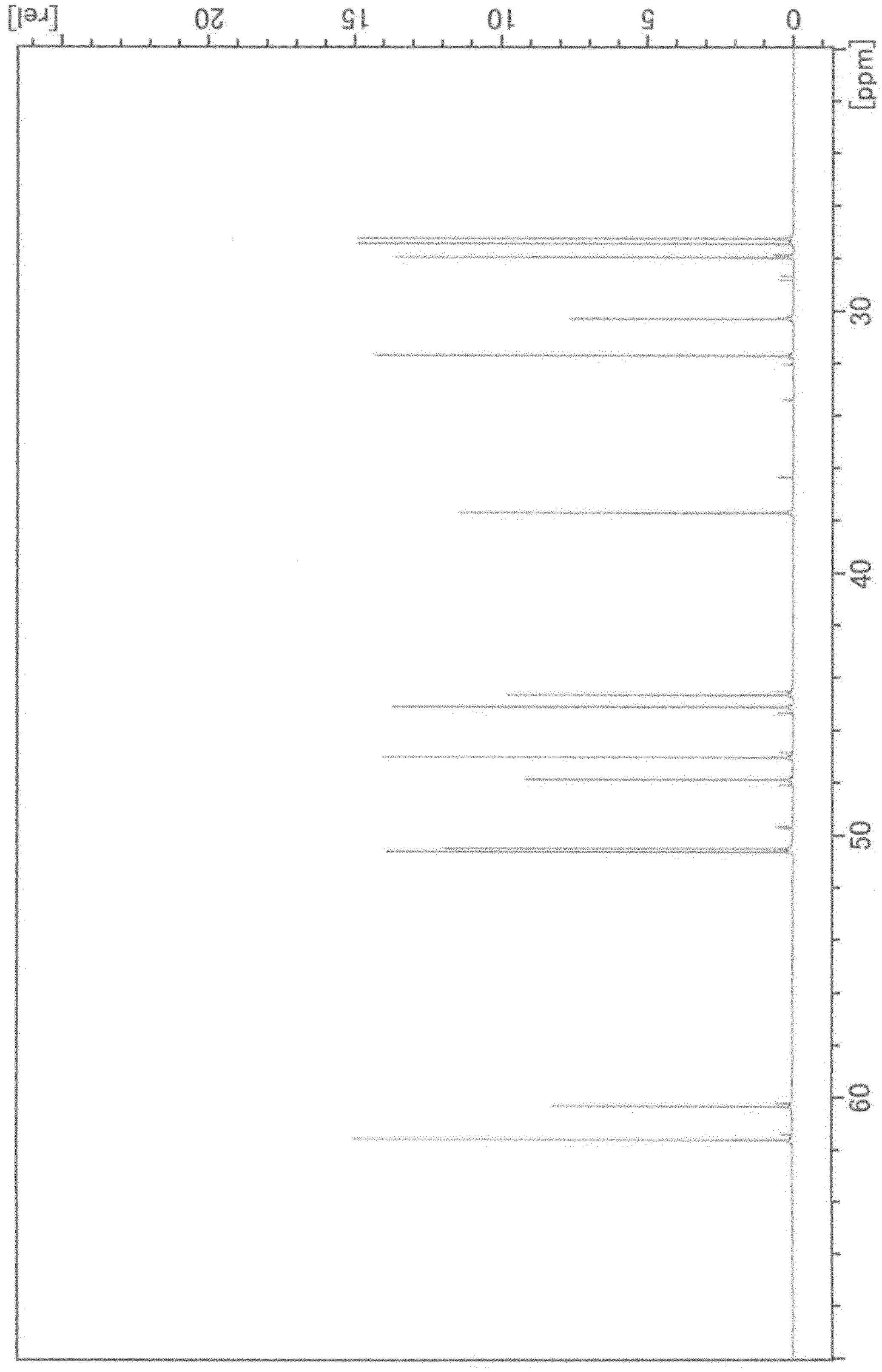


FIG. 3

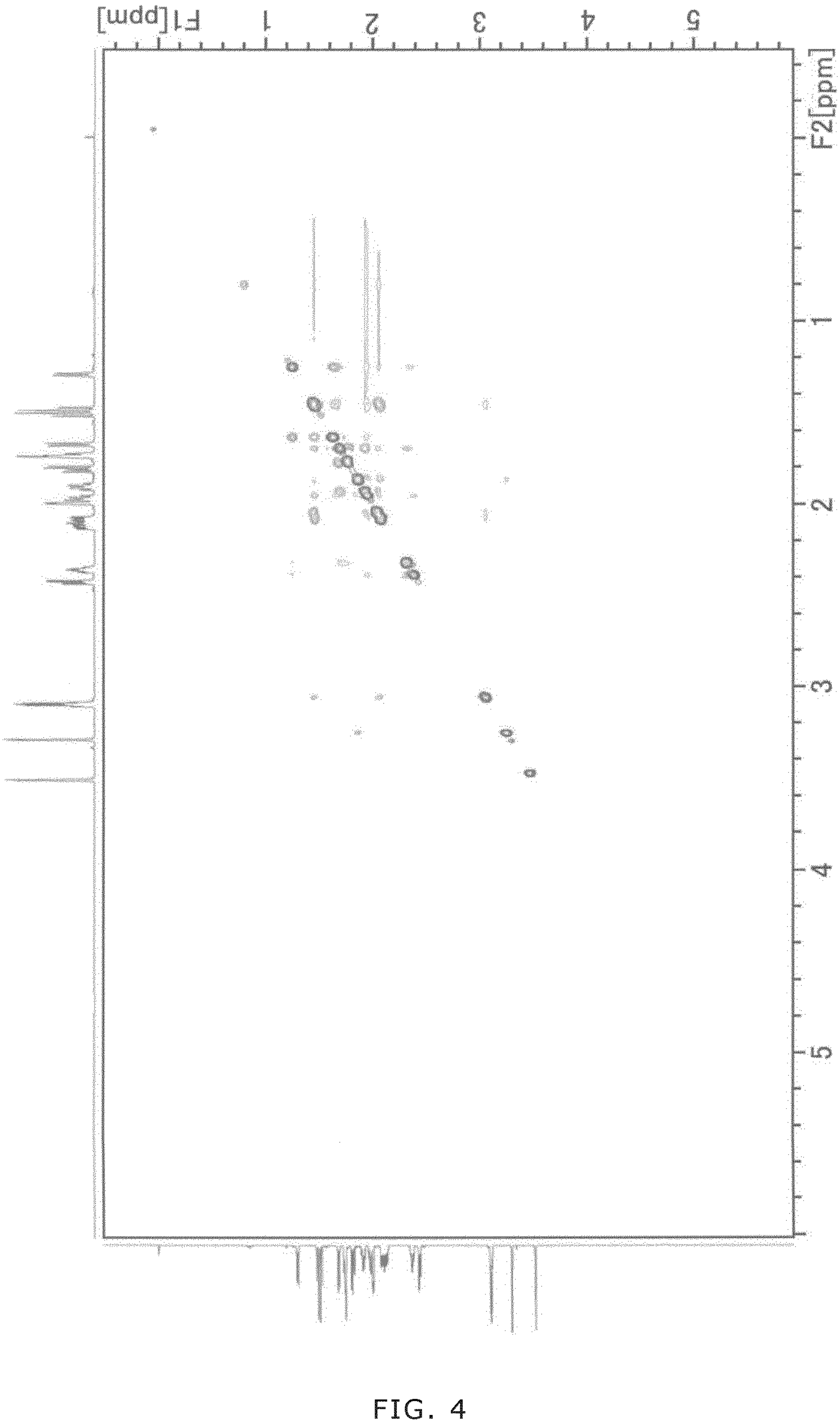


FIG. 4

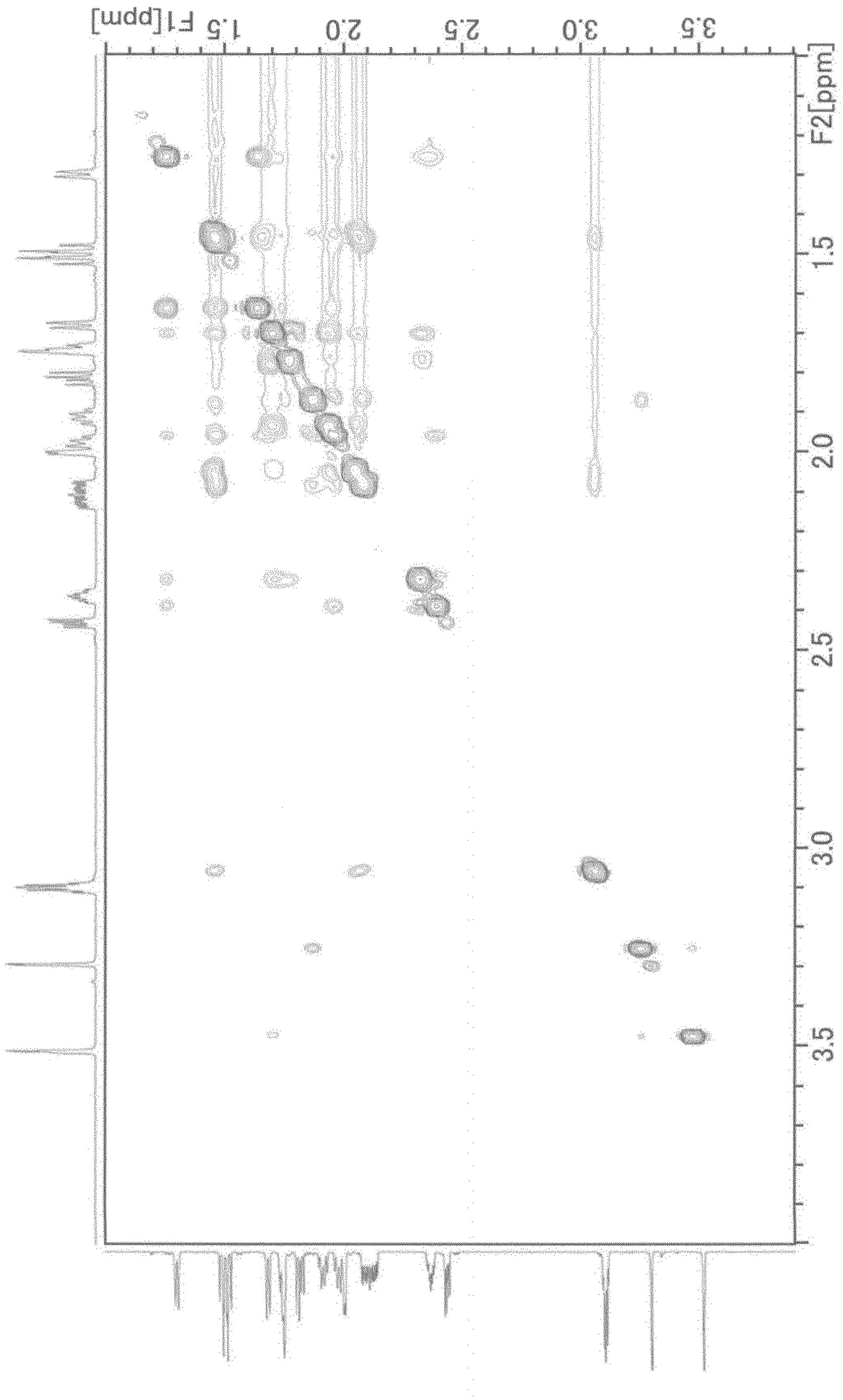


FIG. 5

**INTERNATIONAL SEARCH REPORT**

International application No. PCT/JP2021/012523

A. CLASSIFICATION OF SUBJECT MATTER
C08G 59/02(2006.01)i; C08G 59/18(2006.01)i; C07D 493/04(2006.01)i
FI: C07D493/04 CSP; C08G59/18; C08G59/02
According to International Patent Classification (IPC) or to both national classification and IPC

B. FIELDS SEARCHED

Minimum documentation searched (classification system followed by classification symbols)
C08G59/02; C08G59/18; C07D493/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2017/164238 A1 (JX ENERGY CORPORATION) 28 September 2017 (2017-09-28) entire text, in particular, paragraphs [0002], [0008], [0018], [0027], [0028], [0094], [0096], [0221]-[0233] | 1-12<br>1-12 |
| X<br>Y | WO 2017/213206 A1 (JXTG NIPPON OIL & ENERGY CORPORATION) 14 December 2017 (2017-12-14) entire text, in particular, paragraphs [0002], [0008], [0009], [0031]-[0085], [0087]-[0089], [0094]-[0102], fig. 4 | 1-12<br>1-12 |
| Y | WO 2018/181857 A1 (JXTG NIPPON OIL & ENERGY CORPORATION) 04 October 2018 (2018-10-04) paragraphs [0059], [0068]-[0076] | 1-12 |
| Y | WO 2018/105743 A1 (JXTG NIPPON OIL & ENERGY CORPORATION) 14 June 2018 (2018-06-14) paragraphs [0089], [0111]-[0119] | 1-12 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 May 2021 (11.05.2021) | 18 May 2021 (18.05.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/012523

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2019/083003 A1 (JXTG NIPPON OIL & ENERGY CORPORATION) 02 May 2019 (2019-05-02) paragraphs [0058], [0071]-[0079] | 1-12 |

Form PCT/ISA/210 (continuation of second sheet) (January 2015)

**INTERNATIONAL SEARCH REPORT**
Information on patent family members

International application No.
PCT/JP2021/012523

| Patent Documents referred in the Report | Publication Date | Patent Family | Publication Date |
|---|---|---|---|
| WO 2017/164238 A1 | 28 Sep. 2017 | EP 3434709 A1 paragraphs [0002], [0008], [0020], [0029], [0030], [0096]-[0099], [0103]-[0105], [0445]-[0459] | |
| WO 2017/213206 A1 | 14 Dec. 2017 | US 2019/0161494 A1 paragraphs [0002], [0010]-[0013], [0056]-[0111], [0113]-[0117], [0122]-[0132] EP 3476849 A1 | |
| WO 2018/181857 A1 | 04 Oct. 2018 | US 2020/0024426 A1 paragraphs [0126]-[0132], [0155]-[0168] | |
| WO 2018/105743 A1 | 14 Jun. 2018 | US 2020-0095422 A1 paragraphs [0068], [0069], [0077]-[0084] EP 3553129 A1 | |
| WO 2019/083003 A1 | 02 May 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP S49126658 A
- JP 2008031424 A